# EUROPEAN PATENT SPECIFICATION

(11) **EP 4 097 733 B1**
(45) Date of publication and mention of the grant of the patent: **28.10.2026**
(21) Application number: 21703611.0
(22) Date of filing: 26.01.2021
(51) Int. Cl.: G16H 20/13, G06Q 30/02, G07F 11/00, G16H 40/00

(54) **HEALTCHCARE ECOSYSTEM**
GESUNDHEITSVERSORGUNGSÖKOSYSTEM
ÉCOSYSTÈME DE SOINS DE SANTÉ

(30) Priority: 30.01.2020 GB 202001296
(43) Date of publication of application: 07.12.2022
(73) Proprietor: Clinova Limited, Southampton SO18 2RZ (GB)
(72) Inventor: KARIM, Arsalan, Shipley BD18 4AR (GB); EBUBEDIKE, Charles, London SW16 2TT (GB)
(74) Representative: Page White Farrer
(86) International application number: PCT/EP2021/051741
(87) International publication number: WO 2021/151883

(56) References cited:
- WO-A1-2018/209319
- CN-A- 109 390 044
- CN-A- 110 097 406
- CN-B- 102 622 700
- CN-B- 104 867 357
- KR-A- 20160 038 661
- KR-A- 20180 047 226
- KR-A- 20200 004 035
- US-A1- 2014 244 307

## Description

### Technical Field

The present disclosure relates to a healthcare ecosystem. More particularly the present disclosure relates to a healthcare ecosystem for connecting consumers with healthcare professionals such as pharmacists, and for enabling those consumers to get access to consumer healthcare products in a way that is most convenient for the consumer and the pharmacist. As will be discussed in more detail below, there are disclosed many new aspects in the proposed healthcare system, including the choice of information that consumers can receive and advice that is provided to consumers. The healthcare ecosystem also includes a payment system including a proprietary currency (CAIDR COIN), enabling consumers and local authorities to securely make payments and purchases, and for those payments to be received and tracked by pharmacies and local authorities. Within the healthcare ecosystem convenient and efficient means for providing products to consumers are disclosed, including a drone delivery system. Pharmacies may subscribe to be part of the disclosed healthcare ecosystem. Additionally, via a disclosed mobile application (CAIDR), consumers may enter biometric details and have their health tracked and/or predicted. The information provided may also enable gaps in the marketplace to be identified and filled.

### Background

Consumer healthcare and illness prevention are challenges facing today's society. For a number of reasons it may be difficult for a person to get the information (or indeed accurate and concise information) that they need in order to make informed decisions. It can also be difficult for a person to obtain the right product, even when they know what it is that they need, for example due to the closure of local pharmacies or because of long distances between pharmacies. With the move towards a cashless society, the traditional methods of payment for medical products may also be inconvenient for the consumer as well as the vendor (e.g. a pharmacist).

CN 109 390 044 A relates to a prescription-based medicine purchase method, device, electronic device and storage medium.

CN 102 622 700 B relates to a medicine shopping guide system for a medicine dispensing machine. CN110097406A discloses a medical service resource optimisation system based on multi-pharmacy cooperation, centred on a "supply chain intelligent panel" with a virtual drug library and optimisation algorithms. A context-awareness module acquires patient location, weather and time data and, together with historical prescription and inventory data, predicts demand and determines optimal production, ordering and stock transfers between pharmacies. For individual patients, the system lists reachable medicine pick-up points (pharmacies) and ranks them according to factors such as walking/bus/metro/self-drive time, stock availability and distance to other landmarks, enabling the patient to choose a preferred pick-up location.

### Summary

The invention is defined by the appended claims. According to a first aspect disclosed herein, there is provided a system comprising: a server arranged to receive information from a user device of a user, and to use the received information to process a request for a product to be made available to the user; two or more apparatus in communication with the server; the server arranged to determine at least one of the two or more apparatus to make the product available to the user, and to provide an indication to the user of a selection of the at least one of the two or more apparatus, the server arranged to determine the at least one of the two or more apparatus to make the product available to the user based at least in part on location information, the location information comprising location information of the user and location information of the two or more apparatus; the server arranged to select at least one of the two or more apparatus based on information of an ability of the user to travel, wherein the information of an ability to travel is based upon one or more of: terrain information; or geological information; at least one of the two or more apparatus comprises a drone arranged to deliver the product to the user, wherein the server is arranged to select the drone to deliver the product to the user when travel of the user to another of the two or more apparatus is determined as difficult or not possible, based on the terrain information and/or the geological information; and wherein the product comprises a consumer healthcare product selected from a plurality of available consumer healthcare products using a diagnostics engine for determining one or more medical symptoms of the user, wherein the diagnostics engine is comprised in one or more of: the server; the user device of the user.

According to an example, the drone is arranged to deliver the product to the user from a repository of products and preferably wherein the repository of products comprises a pharmacy.

According to an example, at least one of the two or more apparatus comprises a machine arranged to dispense the product to the user and preferably wherein the machine comprises a vending machine.

According to an example, at least one of the two or more apparatus comprises a robot arranged to provide the product for the user.

According to an example, the system comprises a payment system.

According to an example, the payment system utilises a proprietary currency, and preferably wherein the payment system enables an authority to track payments made via the payment system.

According to an example, the system comprises an interactive tool enabling a user to perform one or more actions.

According to an example, the one or more actions comprises: a health assessment; a drug interaction test to determine whether there are any drugs that the user is allergic to; a sports test; tracking menstrual cycle; tracking weight; tracking sleep pattern; providing one or more bio-markers for assessment.

According to an example, the interactive tool is configured to utilise a facial recognition algorithm to determine conditions of one or more of: skin condition; depression; stress; high/low blood pressure of a user.

According to an example, the system comprises a research and development engine which is configured to, over time, learn efficacy of one or more products by one or more of: sales data; user reviews; surveys.

According to an example, the system is configured to enable a live video-chat between a user and a pharmacist.

According to a second aspect there is provided a server comprising: a controller arranged to receive information from a user device of a user, and to use the received information to process a request for a product to be made available to the user; the controller arranged to determine at least one of two or more apparatus to make the product available to the user, and to provide an indication to the user of a selection of the at least one of the two or more apparatus, the controller arranged to determine the at least one of the two or more apparatus to make the product available to the user based at least in part on location information, the location information comprising location information of the user and location information of the two or more apparatus; the controller arranged to select at least one of the two or more apparatus based on information of an ability of the user to travel, wherein the information of an ability to travel is based upon one or more of: terrain information; or geological information; at least one of the two or more apparatus comprises a drone arranged to deliver the product to the user, wherein the controller is arranged to select the drone to deliver the product to the user when travel of the user to another of the two or more apparatus is determined as difficult or not possible, based on the terrain information and/or the geological information; and wherein the product comprises a consumer healthcare product selected from a plurality of available consumer healthcare products using a diagnostics engine for determining one or more medical symptoms of the user, wherein the diagnostics engine is comprised in one or more of: the server; the user device of the user.

According to a third aspect there is provided a method comprising: receiving information from a user device of a user; using the received information to process a request for a product to be made available to the user; determining at least one of two or more apparatus to make the product available to the user, the determining the at least one of the two or more apparatus to make the product available to the user based at least in part on location information, the location information comprising location information of the user and location information of the two or more apparatus; providing an indication to the user of a selection of the at least one of the two or more apparatus; selecting at least one of the two or more apparatus based on information of an ability of the user to travel, wherein the information of an ability to travel is based upon one or more of: terrain information; or geological information; wherein at least one of the two or more apparatus comprises a drone arranged to deliver the product to the user and selecting the drone to deliver the product to the user when travel of the user to another of the two or more apparatus is determined as difficult or not possible, based on the terrain information and/or the geological information; and wherein the product comprises a consumer healthcare product and selecting the product from a plurality of available consumer healthcare products using a diagnostics engine for determining one or more medical symptoms of the user, wherein the diagnostics engine is comprised in one or more of: a server; the user device of the user.

According to a fourth aspect there is provided a computer program comprising computer executable instructions which when run on one or more processors perform: receiving information from a user device of a user; using the received information to process a request for a product to be made available to the user; determining at least one of two or more apparatus to make the product available to the user, the determining the at least one of the two or more apparatus to make the product available to the user based at least in part on location information, the location information comprising location information of the user and location information of the two or more apparatus; providing an indication to the user of a selection of the at least one of the two or more apparatus; selecting at least one of the two or more apparatus based on information of an ability of the user to travel, wherein the information of an ability to travel is based upon one or more of: terrain information; or geological information; wherein at least one of the two or more apparatus comprises a drone arranged to deliver the product to the user and selecting the drone to deliver the product to the user when travel of the user to another of the two or more apparatus is determined as difficult or not possible, based on the terrain information and/or the geological information; and wherein the product comprises a consumer healthcare product and selecting the product from a plurality of available consumer healthcare products using a diagnostics engine for determining one or more medical symptoms of the user, wherein the diagnostics engine is comprised in one or more of: a server; the user device of the user.

### Brief Description of the Drawings

To assist understanding of the present disclosure and to show how embodiments may be put into effect, reference is made by way of example to the accompanying drawings in which:
Figure 1 schematically shows a healthcare ecosystem according to an example;
Figure 2 schematically shows a drone delivery system according to an example;
Figure 3 schematically shows an app. based interaction between a user and a pharmacist according to an example;
Figures 4 to 23 schematically show app. screens according to various examples;
Figure 24 shows schematically a machine according to an example;
Figure 25 shows schematically a memory structure of the machine of Figure 24, according to an example.
Figure 26 is a flowchart of a method according to an example.
Figure 27 schematically shows a system according to an example.
Figure 28 schematically shows features of an apparatus such as a mobile device, according to an example.

### Detailed Description

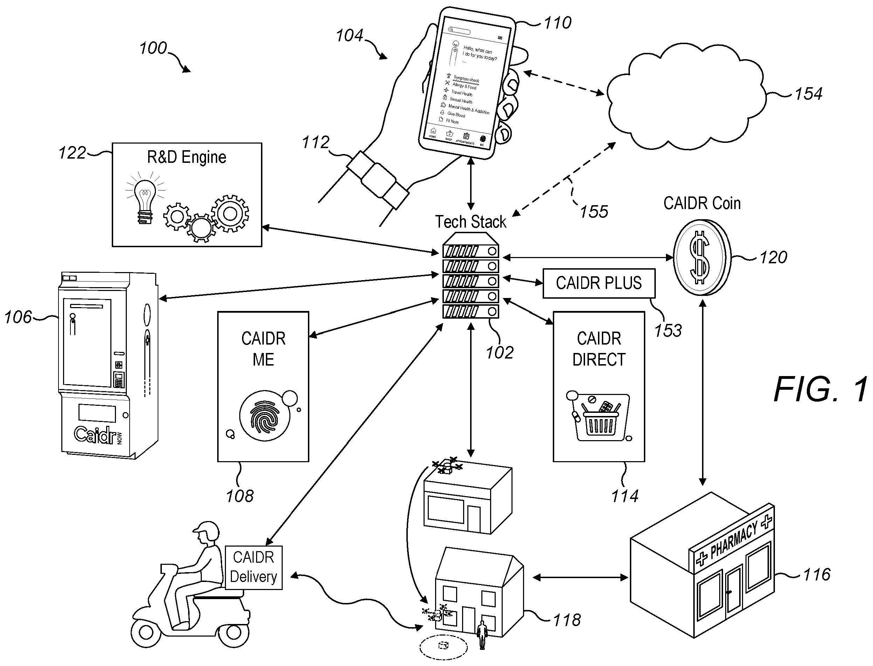

Figure 1 schematically shows a healthcare ecosystem 100 according to an example.

According to an example, the product comprises a consumer healthcare product selected from a plurality of available consumer healthcare products using a diagnostics engine for determining one or more medical symptoms of the user.

According to an example, the diagnostics engine is comprised in one or more of: the server; the user device of the user.

According to an example, the system comprises a healthcare system.

According to a third aspect there is provided a server comprising: a controller arranged to receive information from a user device of a user, and to use the received information to process a request for a product to be made available to the user; the controller arranged to determine at least one of two or more apparatus to make the product available to the user, and to provide an indication to the user of the selection of the at least one of the two or more apparatus.

According to a fourth aspect there is provided a method comprising: receiving information from a user device of a user; using the received information to process a request for a product to be made available to the user; determining at least one of two or more apparatus to make the product available to the user; and providing an indication to the user of the selection of the at least one of the two or more apparatus.

According to a fifth aspect there is provided a computer program comprising computer executable instructions which when run on one or more processors perform: receiving information from a user device of a user; using the received information to process a request for a product to be made available to the user; determining at least one of two or more apparatus to make the product available to the user; and providing an indication to the user of the selection of the at least one of the two or more apparatus.

According to a sixth aspect there is provided a machine for dispensing one or more products, the machine comprising: a controller; a storage area for storing a plurality of products; a display for displaying a user interface; the controller configured to receive an input from a user via the user interface, the input comprising an indication of one or more medical symptoms of the user; the controller configured to process the received indication of one or more medical symptoms of the user, to determine a product suitable for treatment of the one or more medical symptoms; and in response to determining a suitable product, the controller further configured to cause the machine to generate an output.

According to an example, the output comprises dispensing of a suitable product.

According to an example, the output comprises information displayed on the user interface informing a user of where a suitable product can be obtained.

According to an example, the output comprises information that a most suitable product is not available from the machine but another suitable product is available from the machine, and providing a user on the user interface with an option of whether to dispense the another suitable product from the machine.

According to an example, the output comprises information of where the most suitable product is available from.

According to an example, the machine is configured to dispense a product in response to receipt of a valid identifier.

According to an example, the valid identifier comprises a character string or a barcode.

According to an example, the machine is in communication with an external server.

According to an example, the machine is arranged to monitor stock-level information of the machine, and to make the stock-level information available to one or more distributors.

According to an example the machine comprises a base, a top, and a front side extending between the base and the top.

According to an example the machine comprises a recessed portion in the front side of the machine, an outlet for dispensing one or more products being located in the recessed portion.

According to an example the recessed portion extends between the base of the machine and a region proximate to a lower edge of the display.

According to an example the controller is configured to process payments by a proprietary currency.

### Brief Description of the Drawings

To assist understanding of the present disclosure and to show how embodiments may be put into effect, reference is made by way of example to the accompanying drawings in which:
Figure 1 schematically shows a healthcare ecosystem according to an example;

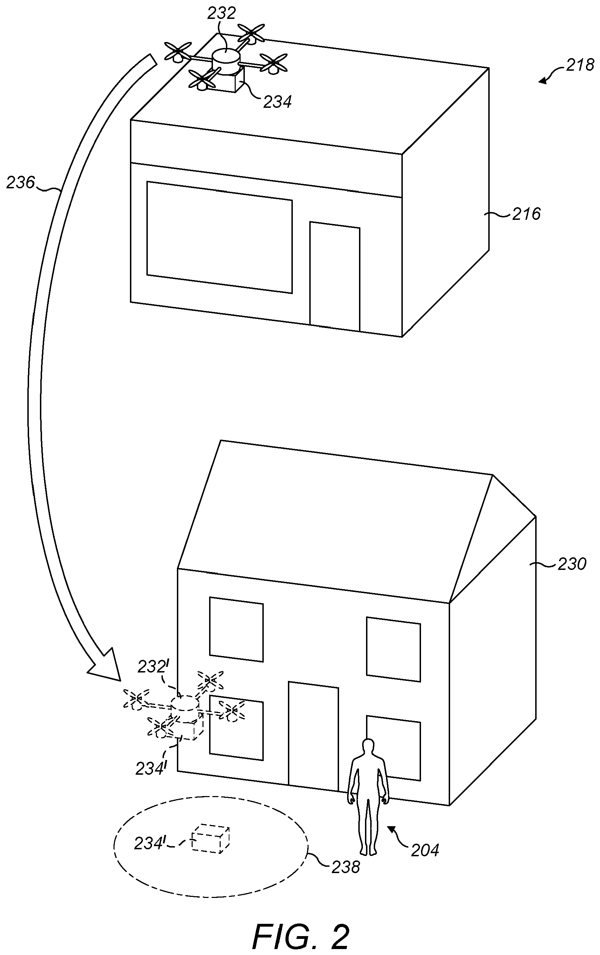

Figure 2 schematically shows a drone delivery system according to an example;

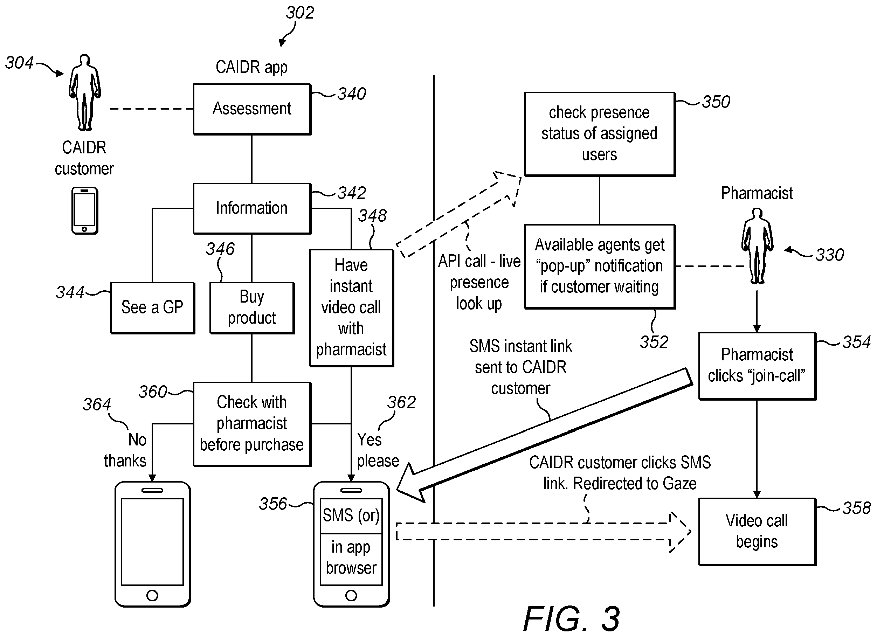

Figure 3 schematically shows an app. based interaction between a user and a pharmacist according to an example;

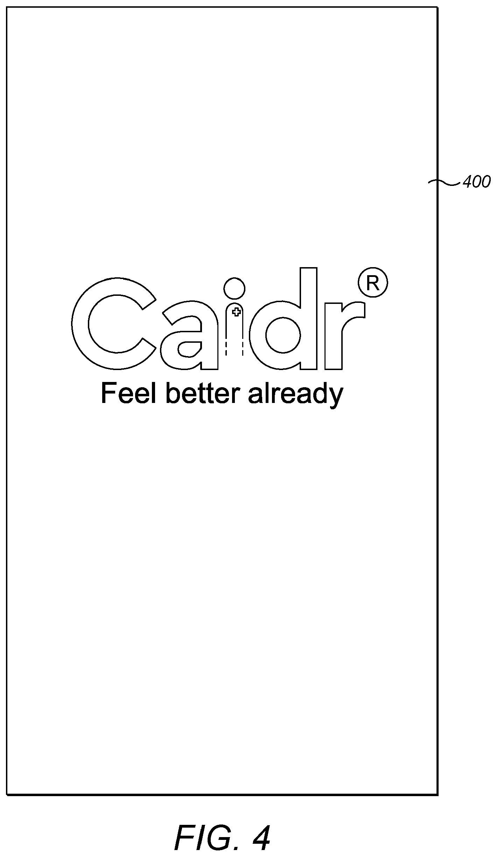

Figures 4 to 23 schematically show app. screens according to various examples;

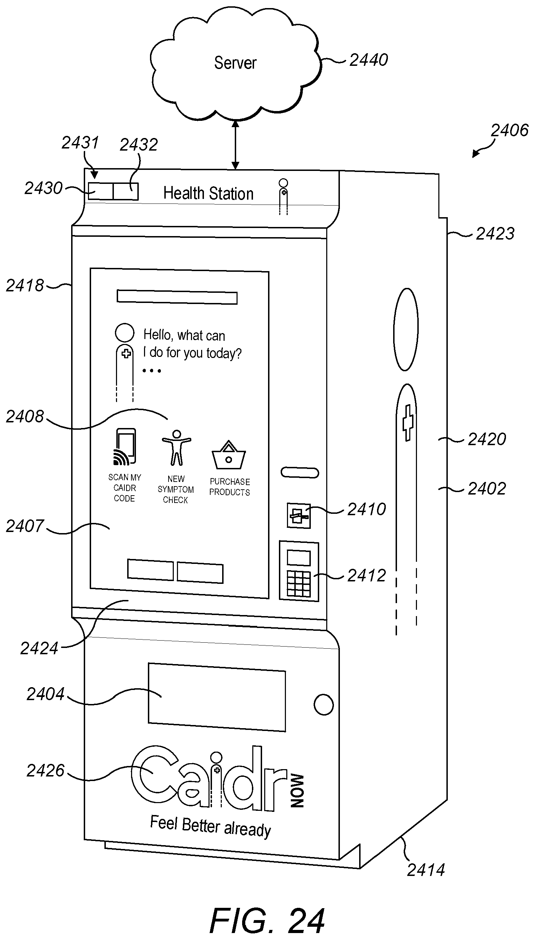

Figure 24 shows schematically a machine according to an example;

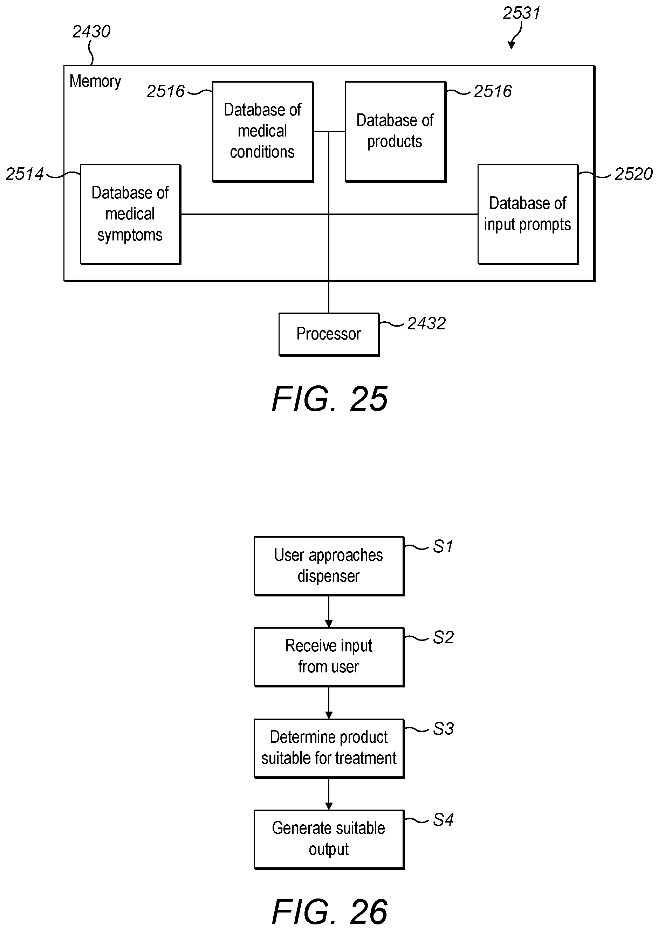

Figure 25 shows schematically a memory structure of the machine of Figure 24, according to an example.
Figure 26 is a flowchart of a method according to an example.

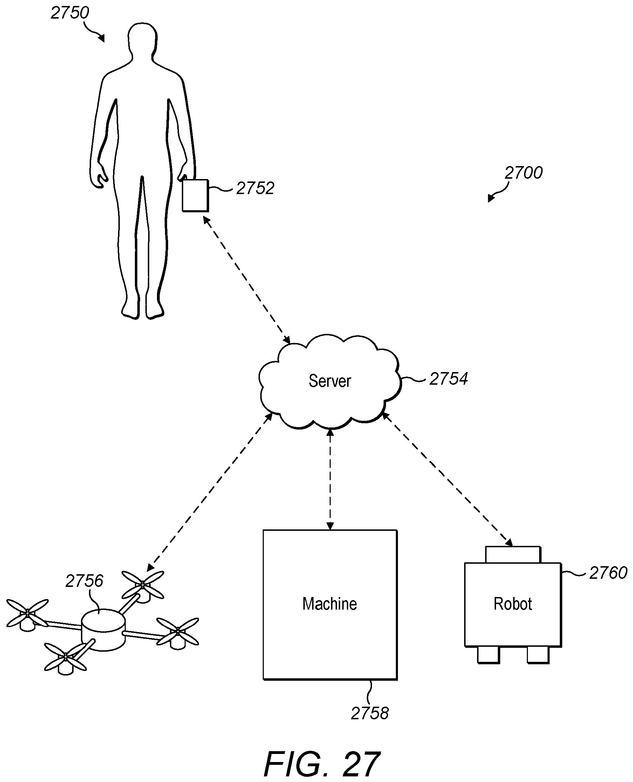

Figure 27 schematically shows a system according to an example.

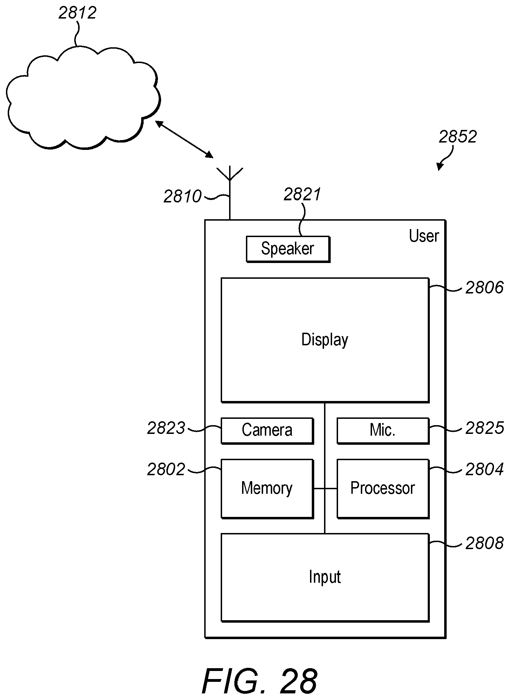

Figure 28 schematically shows features of an apparatus such as a mobile device, according to an example.

### Detailed Description

Figure 1 schematically shows a healthcare ecosystem 100 according to an example.

"CAIDR" shown at 102 represents a platform or app via which a user or consumer 104 may interact with the system 100. CAIDR (www.caidr.co.uk) is a digital healthcare app which helps users assess ailments. In examples, the CAIDR app is downloadable from, for example, Apple iTunes^{®} and Google Play^{®}. Via the CAIDR app 102 a user can be assisted with determining a medical ailment and be provided with advice and guidance of how to deal with the ailment, as described in more detail in GB2561156A and WO2018/171919A1. For example a user 104 may be able to interact with the CAIDR app via a smartphone 110 or other computing device of the user. In some examples the user 104 can interact with the CAIDR app 102 using voice and/or video, for example for interacting with a doctor or a pharmacist, or in some examples for interacting with a virtual assistant. In some examples there is provided a "black-out" feature which enables a user to select a time when the functionalities of the user's device 110 are reduced or completely removed. In some examples the black-out functionality is implemented via the CAIDR app. In some examples the black-out functionality is enabled once a user has entered a serial number of their phone and/or their phone number via the app. In some examples the user can select times of day or time ranges during which the black-out feature is active. This can help a user alleviate harmful effects of over-use of their device. In some examples a user can implement the black-out on phones other than their own (once the necessary permissions are in place). For example, a parent may be enabled to black-out a device of their child.

"CAIDR NOW' shown at 106 represents a machine or a system or array of machines from which user 104 can obtain one or more products for treating ailments. Such machines may be located at places where typically there would not be a pharmacy, such as on a ship, on an oil rig, on a mountain etc. The machines could of course also be located at other locations such as universities, in GPs' surgeries, in hospitals etc. The CAIDR^{®} NOW machines effectively make a pharmacy available in locations where there may not already be a pharmacy, which can result in a vast increase in the number of pharmacies effectively available to users. The machines are discussed in more detail below, for example with respect to Figure 24.

"CAIDR ME" shown at 108 is an interactive tool (e.g. accessed on or via the CAIDR app) that enables user 104 to enter details via the CAIDR platform 102, and perform one or more further actions. For example, the user may perform any one or more of: a drug interaction test to determine whether there are any drugs that the user is allergic to; a sports test (e.g. for an athlete); track their menstrual cycle; track their fertility; track their weight; track their sleep pattern. In some examples the CAIDR ME platform 108 can automatically obtain information from a fitness tracker 112 and/or the user equipment 110 of the user. According to some examples CAIDR ME comprises a functionality (CAIDR Select) which determines whether any recommended products (or products which would otherwise be recommended) are safe, appropriate and effective based on a user's condition and/or profile (e.g. pregnant, elderly, a sports person who cannot consume banned substances etc.).

Via CAIDR ME 108 the user 104 may also be able to provide one or more biomarkers for assessment. For example, a user can cough in to a microphone of their device 110, which cough sample is assessed by the CAIDR app to determine what type of cough it is (e.g. dry, chesty etc.), which can help determine what kind of ailment the user has. For example the CAIDR app may employ a machine learning algorithm (such as a convolutional neural network (CNN)) to compare the supplied cough sample with a library of stored cough sounds and associated ailments. Similarly a user can provide an image (for example via a camera of their device 110) of a visible ailment (e.g. a skin condition), which image is then compared to a library of stored images and associated ailments (e.g. skin conditions), so that relevant information can be provided to the user 104. In some examples a facial recognition algorithm is employed to help determine conditions of depression, stress, high/low blood pressure etc. of the user 104. The provided biomarkers are in some examples used to predict an age of the user 104. Based on the biomarkers, in some examples notifications are provided to the user 104 (e.g. daily notifications). The notifications may for example provide health advice and/or motivational messages to the user. Where a user is tracking their fertility, the notifications may also inform the user of when they are at their most fertile. The notifications may additionally or alternatively comprise dosage reminders. In some examples the dosage reminders are also automatically entered in to the user's own online calendar (e.g. Google^{®} calendar, Outlook^{®}, iCal^{®} etc.).

In some examples (for example also via CAIDR ME) the user 104 can complete a health assessment. In some examples, a video of a doctor or pharmacist (who may in some examples be virtual) is provided on the CAIDR app for taking the user 104 through the assessment. In some examples the user 104 can select a language of the doctor or pharmacist, making the assessment easier and improving the likelihood of receiving accurate answers from the user 104. In some examples the assessment comprises a food allergy assessment. In some examples the assessment may require a physical test to be carried out (e.g. a blood test). Where this is the case, a blood testing kit may be sent in the post (or collected from an agreed location) by the user 104, who takes a blood sample and then sends the blood sample to a laboratory for testing (e.g. for testing against certain blood types). Once the test has been carried out, the laboratory can upload the results in to the CAIDR app for assessment by the doctor or pharmacist (and for viewing by the user 104). In other words the entire assessment (even if requiring a physical test such as a blood test) and its results can be administered via the CAIDR app.

"CAIDR^{®} DIRECT" 114 is a platform that provides direct delivery of products to the user 104. For example CAIDR^{®} DIRECT may be accessed via the CAIDR app on the user's smartphone, enabling the user 104 to buy one or more products for direct delivery to their home.

A pharmacy is schematically shown at 116. The pharmacy 116 may be a physical pharmacy, for example located in a town or city. The pharmacy 116 is communicatively connected to CAIDR drone delivery system 118. The CAIDR drone delivery system comprises a drone system (i.e. comprises one or more drones) for delivering products to the pharmacy 116, and/or for delivering products from the pharmacy 116 to the user 104. This means that in some examples the pharmacy 116 does not need to store lots of products, and can have the products flown in on an as-needed basis. For example the user 104 may order a product via the CAIDR app, and have the product delivered for collection at their pharmacy 116. Also, the user 104 can have products delivered from the pharmacy 116 directly to a place of choosing of the user 104 (e.g. the user's home or workplace). The drone delivery system 118 is described in more detail with respect to Figure 2.

A payment system is schematically shown at 120. In some examples the system 120 utilises blockchain technology. System 120 may be used for tracking products and/or payments. In some examples the system comprises a proprietary CAIDR blockchain. In some examples the system 120 utilises an existing blockchain. In some examples the system 120 provides a proprietary currency for payment exclusively via the CAIDR app or platform. For example the currency may be called "CAIDR coin". In some examples the CAIDR coin comprises a cryptocurrency. In other examples the CAIDR coin may be backed by a traditional currency, such as pounds sterling, dollar or Euro etc. The user 104 may pay for one or more products on the CAIDR app using the CAIDR coin. The pharmacy 116 may also receive payment via the payment system 120. In some examples users may be provided with CAIDR coins, for example by a local authority or health service. This gives the local authority or health service confidence in knowing that the user can only spend the CAIDR coins via the CAIDR app. (e.g. only on healthcare products). The electronic nature of the CAIDR coin also makes it easy for payments to be tracked and monitored e.g. by the local authority.

In some examples the pharmacy 116 may subscribe to the CAIDR platform in order to take advantage of, for example, CAIDR's users 104, CAIDR drone delivery system 118, and the payment system.

An R&D (research and development) engine is schematically shown at 122. The R&D engine 122 links-in with the CAIDR platform 102. The R&D engine 122 can over time learn what products people need and like. The R&D engine 122 can also learn which products are effective and which are not so effective, or in other words learns efficacy of products. In some examples the R&D engine 122 automatically learns this information (or data) over time e.g. from sales data, reviews etc. Additionally or alternatively the CAIDR platform surveys its users for their opinions of the products they have used. This information can then be fed in to the R&D engine 122. The information from the R&D engine 122 can also be used by pharmacists to learn which products are most effective and therefore learn which products they should stock and/or make available to their customers. In some examples the R&D engine uses the received data to spot gaps in the marketplace and then create or help to create new solutions (e.g. new products) based on the data to improve product selection.

CAIDR PLUS is schematically shown at 153.

In examples, operation of one or more of CAIDR platform 102; R&D engine 122; CAIDR NOW 106; CAIDR ME 108; CAIDR drone delivery system 118; CAIDR DIRECT 114; and payment system 120 are stored and/or controlled by one or more servers shown schematically at 154 (e.g. in the cloud), and may communicate with each other via the Internet as schematically shown by dashed line 155.

Thus for example it may be considered that the server 154 is arranged to receive information from a user device 110 of a user (for example via CAIDR app), and to use the received information to process a request for a product to be made available to the user. The server 154 is then arranged to determine at least one of two or more apparatus (e.g. CAIDR NOW machine 106; CAIDR drone system 118; pharmacy 116) to make the product available to the user, and to provide an indication to the user (e.g. on the CAIDR app) of the selection of the at least one of the two or more apparatus.

Drone delivery system 118 will now be described in more detail. In Figure 2 the system is represented with reference numeral 218. A pharmacy is shown at 216. A drone 232 is shown at the pharmacy 216. In this example a user's designated place (e.g. home or workplace) is shown at 230. When user 204 places an order for a healthcare product via the CAIDR app, the user 204 can select for the product to be delivered to the designated place 230 with drone delivery system 218. In this example the user 204 has ordered product 234 to be delivered. As schematically represented by arrow 236, the drone 234 flies to the user's designated place 230. When outside the user's designated place, the drone (shown at 232') projects a target area 238 such as a circle on to the ground. The drone 232' then drops or places the product 234' in to the target area 238. Once the product 234' has been removed from the target area 238 by the user 204, the drone can return to the pharmacy 216.

Figure 3 schematically shows the way in which a user 304 can interact with CAIDR app 302 (left-hand side of Figure 3) and/or with a pharmacist 330 (right-hand side of Figure 3).

Via the CAIDR app 302 the user 304 can perform an assessment as shown at 340. The assessment may involve answering one or more questions provided by the app. Based on responses from the user 304, the app provides information as shown at 342.

The information could for example be to see a GP 344 or to buy a recommended product 346. Alternatively the information 342 may recommend a video call with a health professional such as a pharmacist, as shown at 348.

When user 304 selects a video call with a pharmacist, than an API (application programming interface) call is made which checks the status presence of assigned users (i.e. assigned pharmacists), as shown at 350.

Available agents (i.e. pharmacists) get a pop-up notification if there is a customer waiting to be spoken to, as shown at 352.

In this example, pharmacist 330 elects to "join-call" as shown at 354.

In some examples this results in a message (e.g. SMS or an in-app message) being sent to the user as shown at 356, to let the user (e.g. customer 304) know that a pharmacist is ready to speak to them. The customer 304 can click a link in the message, which then causes the video call to begin, as shown at 358.

In some examples the customer 304 can, during the video call, check any recommended products with the pharmacist, before purchase. This is shown at 360. The customer can choose whether to continue with the purchase as shown at 362, or decline the purchase as shown at 364.

Some screens (i.e. user interfaces) of the CAIDR app will now be described.

Figure 4 shows a welcome screen or "splash-page" 400 of the CAIDR app.

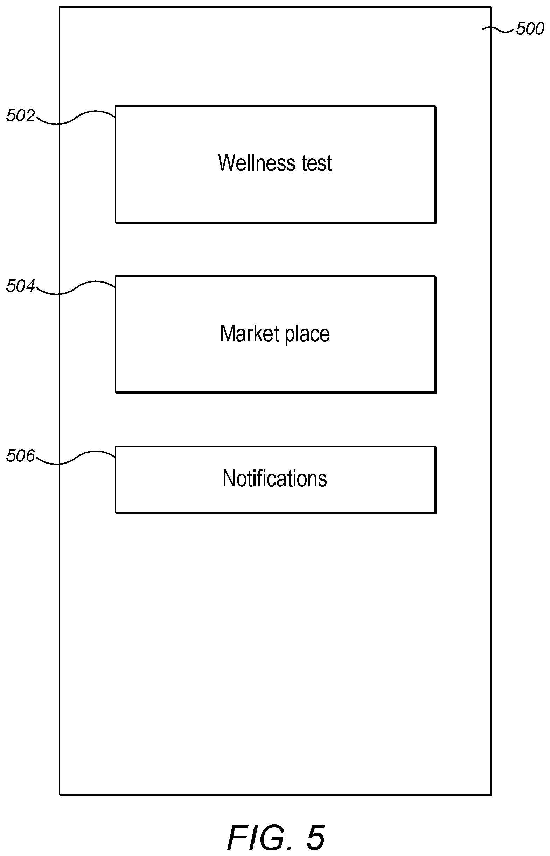

A schematic example of a high level menu 500 of CAIDR is schematically shown in Figure 5.

Via the screen 500, a user can choose to perform a wellness test 502. In some examples the wellness test consists of several tests measuring biometrics based on the user's requirements and needs. According to some examples an essential, baseline or advanced home blood test may be organised via the app as part of the wellness test. Based on how the app is used by the user the wellness test will over time become personalised and interactive to that user by using artificial intelligence (AI) to monitor information provided by / otherwise obtained (e.g. from a fitness tracker) of the user. Each user will receive an in-app notification, for example in notifications area 506, highlighting whether the user will benefit from a wellness test, and general natural ailments and advice to improve the user's health. In some examples the wellness test may test one or more of the following: Glucose; cholesterol; lipid profile; bone profile; uric acid; ferritin; cervical smear (women); vitamin D; vitamin B; lifestyle questions; Body Mass Index; body fat; urine analysis; spinal assessment; lung function test; Electrocardiogram; blood pressure; bowel cancer test; vision test; and fitness test. At least some of these tests may require a physical examination of the user, or at least for samples to be sent by the user for testing. Nevertheless, the storing of results and provision of results back to the user may all be conducted via the CAIDR app.

A market place 504 is also accessible via screen 500. The market place 504 allows a user to search for a designated private healthcare professional of choice based on specific needs of the user. In some examples the app uses live location information of the user (e.g. GPS coordinates). The app may then allow the user to search for the care that they need at their location. The app may also allow the user to book an appointment, for example using the app as a platform.

The notifications area 506 may enable in-app notifications. For example after a product choice or health information, regular, relevant updates may be sent to the user.

According to some examples, one or more products made available via the app may be given a "seal of approval". The seal of approval may be provided for a list of consumer treatments for each ailment where applicable. Such a seal of approval may in some practical examples be known as "CAIDR choice". In some examples the medical team will list the most appropriate CAIDR choice products for each applicable ailment based on affordability, efficacy, safety, availability and consumer feedback. CAIDR choice is aimed at saving each user time and effort with regards over the counter treatments for each self-treatable ailment.

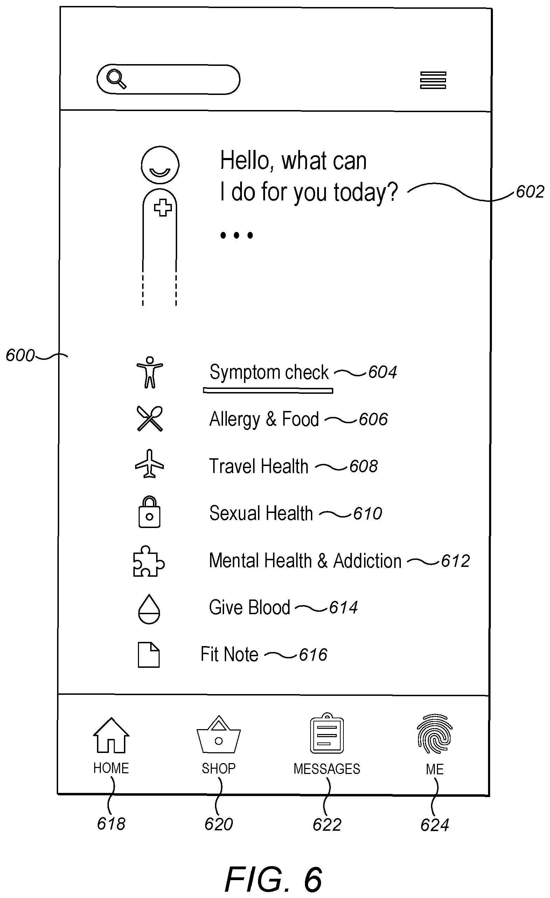

Figure 6 shows a further CAIDR screen 600. The screen 600 prompts a user to choose an option, in this example with the prompt 602 of "Hello, what can I do for you today?". In this example available options are symptom check 604, allergy and food 606, travel health 608, sexual health 610, mental health & addiction 612, give blood 614, fit note 616. In some examples section 606 is an "in-house" section which focuses on patients who have a food or other type of allergy. The most appropriate test for each consumer may be recommended. The travel health section at 608 highlights to consumers precautions that need to be taken when travelling, such as travel advice, travel medication, vaccinations etc. Sexual health section 610 may enable users to answer a list of questions to establish whether they have any sexual health issues. If treatment is required the user may be redirected e.g. to a third party providing relevant services. Section 612 enables a user to explore if they have any mental health or addiction issues, which may be effected through use of one or more algorithms. In the lower part of the screen 600 are further options, including: home 618 to return the user to the homepage; shop 620 via which a user can purchase one or more products; messages 622 via which a user can view messages; and CAIDR ME 624 (i.e. link in with the CAIDR ME section 108 as described with respect to Figure 1).

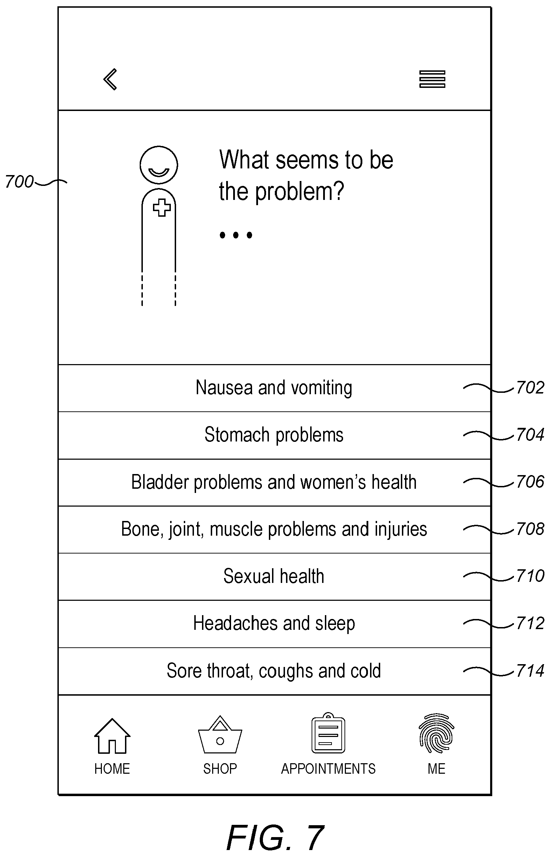

If a user selects symptom check 604, then in some examples they are taken to a follow-on screen 700 as shown in Figure 7. In this screen the user is prompted to enter information of the user's symptoms by means of prompt, "What seems to be the problem?". The user can enter symptoms based on one or more categories, e.g. nausea and vomiting 702; stomach problems 704; bladder problems and women's health 706; bone, joint, muscle problems and injuries 708; sexual health 720; headaches and sleep 712; sore throats, coughs and cold 714..

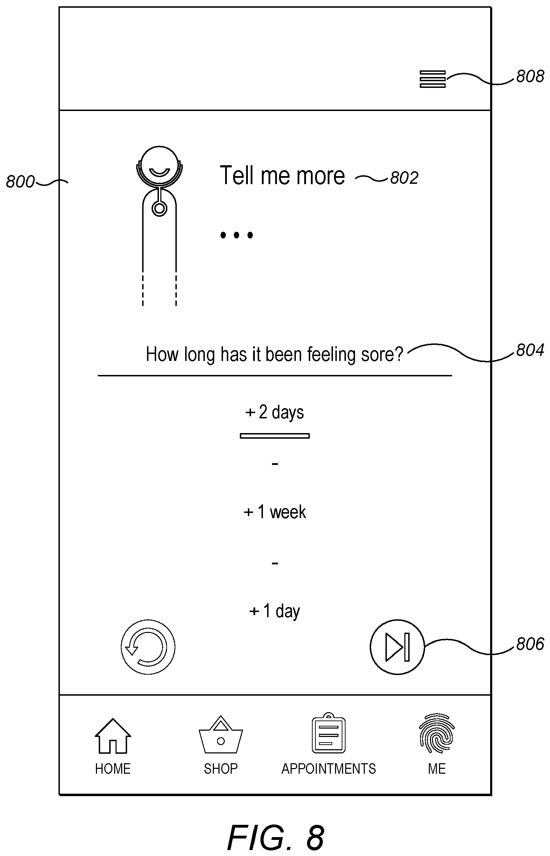

Once the user has entered initial information of their symptoms, the user may then be asked further questions in a tapered style as shown in the screen 800 of Figure 8 i.e. by being asked more specific questions. For example the user is prompted to enter further information with the prompt "Tell me more" 802. The user is then asked to specify how long they've had the symptom for with the prompt 804. In some examples the user can select to move on to the next question or next stage by selecting the icon 806.

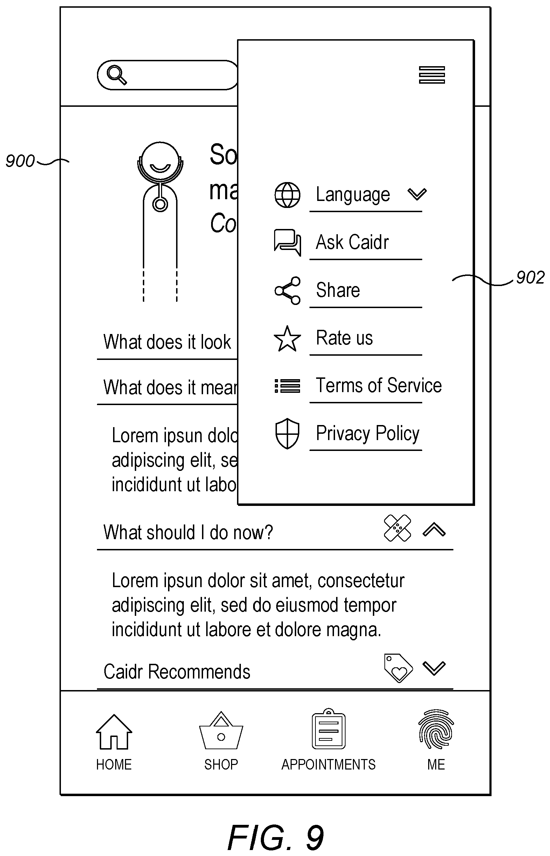

Figure 9 shows a screen 900 with a drop-down list 902. The drop-down list may be selected by selecting the icon or hamburger button 808 shown in Figure 8.

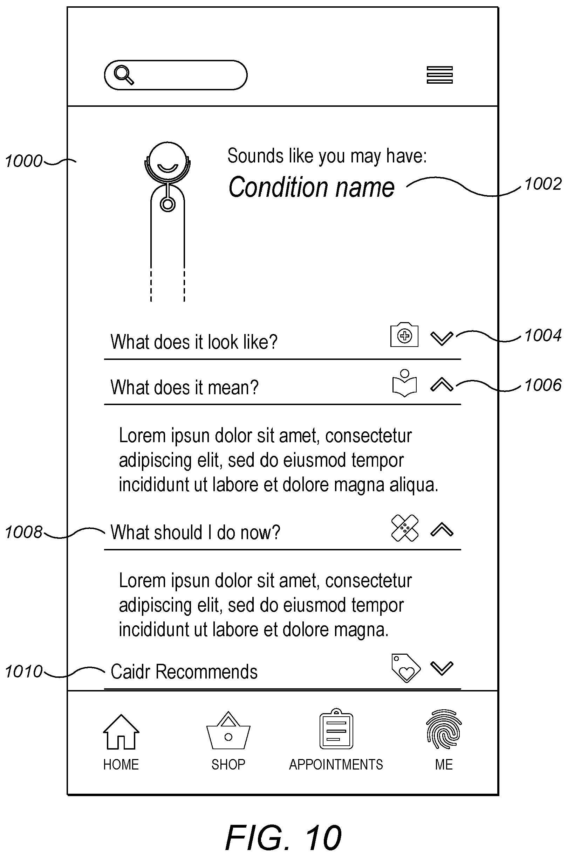

Figure 10 shows a screen 1000 which may be presented to a user once the app has determined the condition that the user is suffering from. For example the screen 1000 may present a name of the condition at 1002 (e.g. headache, blepharitis, hayfever etc.). Further information may be provided to the user about the condition, such as: "what does it look like?" at 1004 (in some examples a visual representation is provided); "what does it mean?" at 1006; "what should I do now?" at 1008. Further information may include a product recommendation by the app for treating the medical condition at 1010. For example the product recommendation may include the "CAIDR choice".

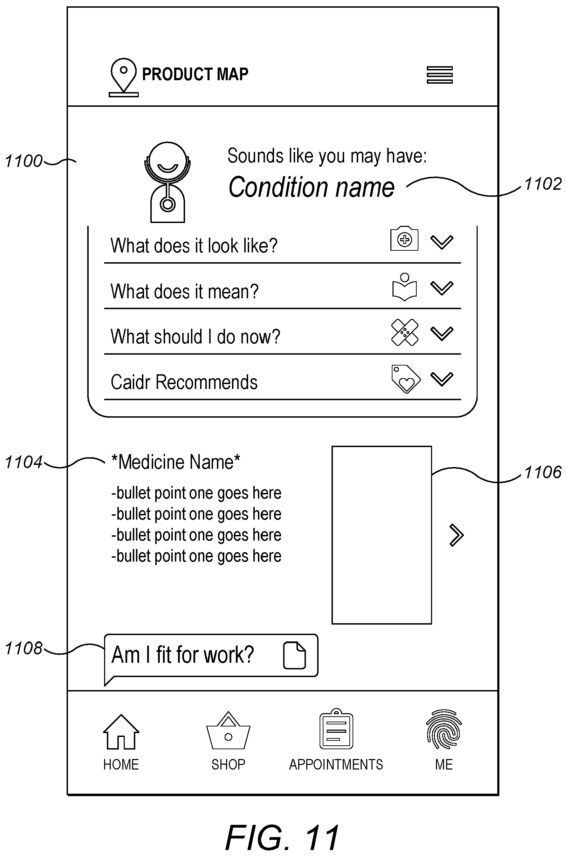

Figure 11 shows a screen 1100 where, in addition to the condition name being provided at 1102, information of a product to treat the condition may also be provided. For example a name of the medicine may be provided at 1104. In some examples an image of the product is also provided as shown schematically at 1106. In some examples a prompt is also provided at 1108, which states "Am I fit for work?". By clicking on prompt 1108 information may then be provided to the user indicating whether the user is determined to be fit enough to attend work.

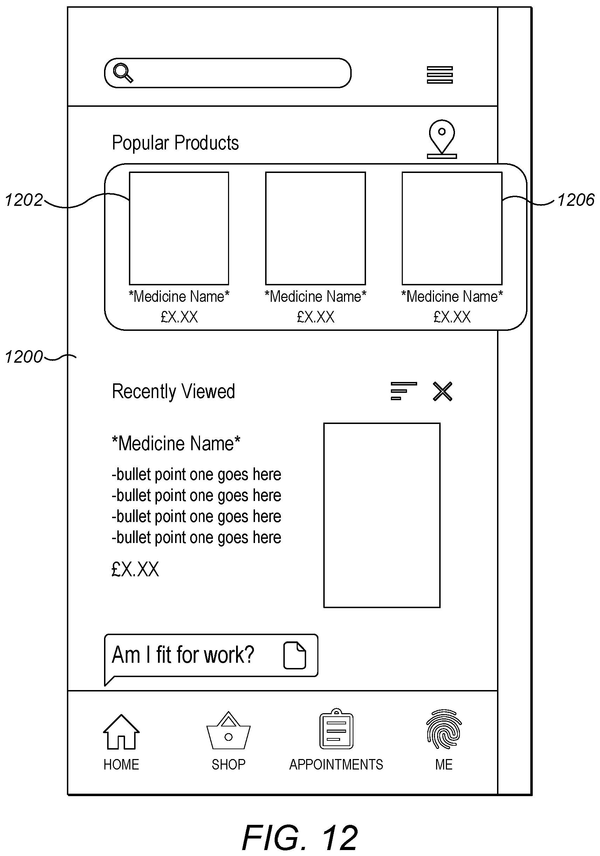

Figure 12 shows a further screen 1200 where a plurality of popular products shown schematically at 1202, 1204, 1206 are provided as recommendations for treating the determined condition. In some examples, for each product a product or medicine name and a price may be provided.

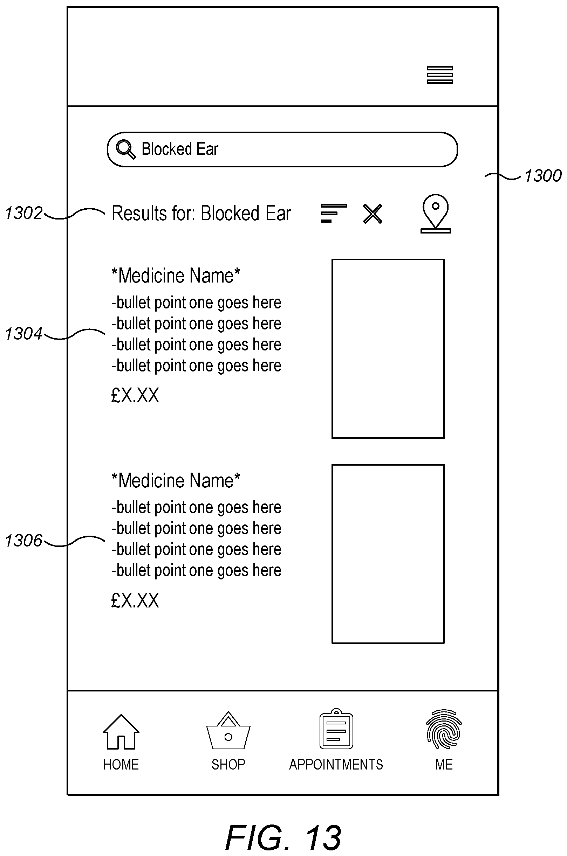

Figure 13 shows a further screen 1300 where in addition to recommended products 1304, 1306, an indication is provided at 1302 indicating what the products are suitable for treating (in the example of Figure 13, "blocked ear").

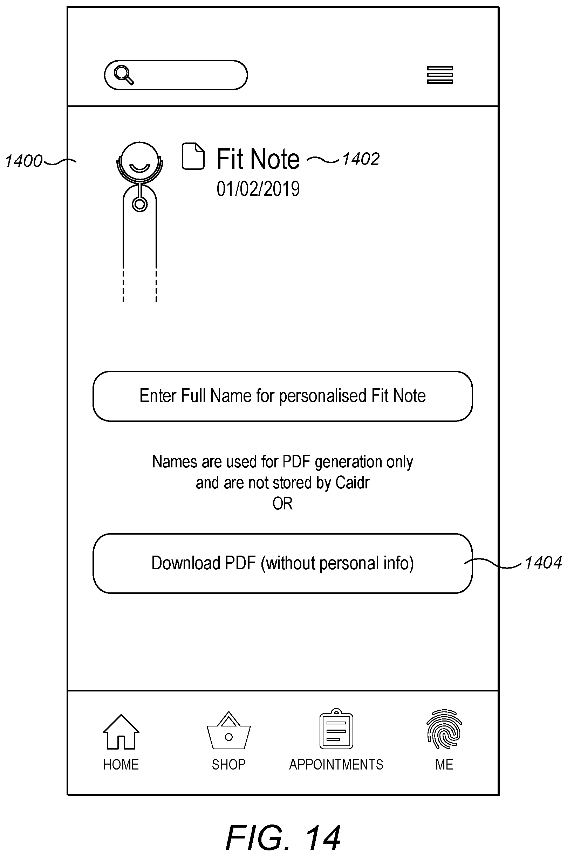

According to some examples a "Fit Note" may be provided, as shown for example in screen 1400 in Figure 14. The fit note may be used by a person to display whether a user is fit or not fit for work, for example. The generation of the fit note may be based on information provided by the user of their medical symptoms. For example by selecting link 1402 the user may be able to download the fit note. Additionally or alternatively, by selecting link 1404 the user may be able to download the fit note in pdf form.

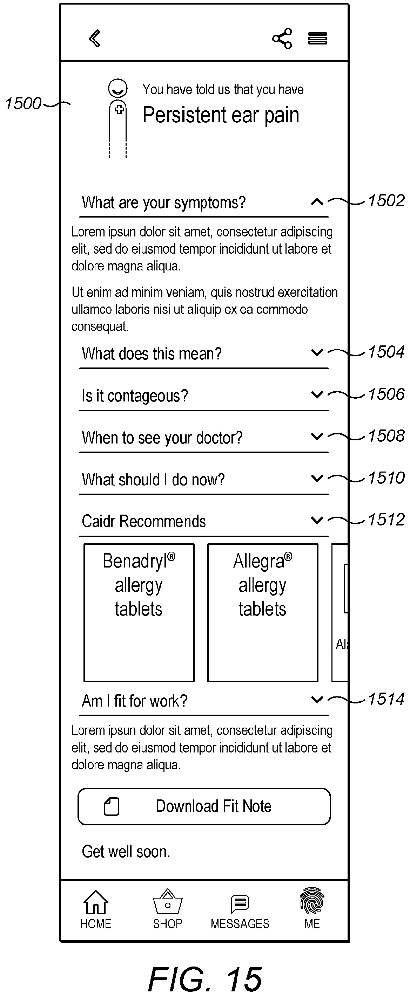

Figure 15 shows an example screen 1500 where it has been determined by the app that the user has persistent ear pain, or the user has informed the app of persistent ear pain. There are a number of drop down information areas on this screen, whereby clicking on a particular area more information is given. These areas include "What are your symptoms" 1502; "What does this mean?" 1504; "Is it contagious?" 1506; "When to see your Doctor" 1508; "What should I do now?" 1510; "CAIDR Recommends" 1512; "Am I fit for work" 1514.

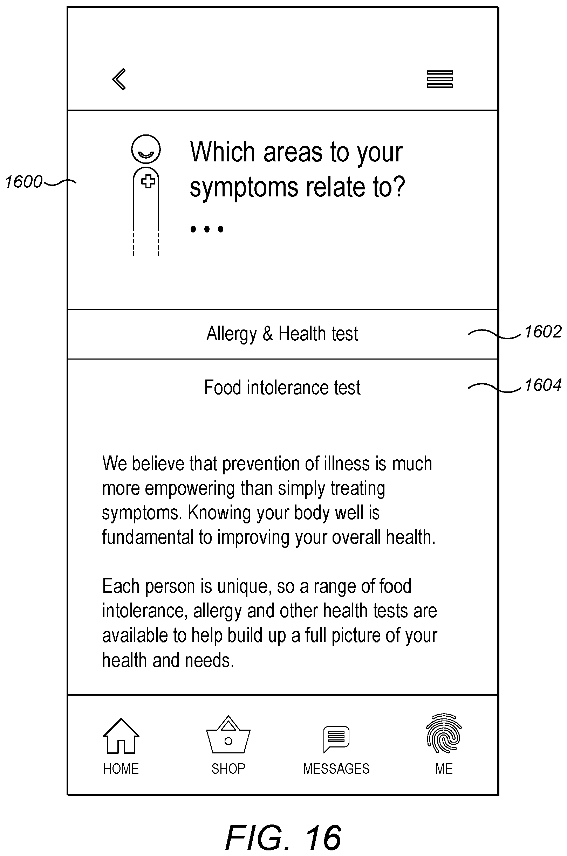

Fig. 16 shows a screen 1600 relating to a health test (e.g. a health test accessible as part of CAIDR ME as previously described). For example the health test may be an Allergy & Health test or a Food intolerance test 1604. Whichever test is chosen, the user may then be provided with appropriate prompts in order to carry out the test in question.

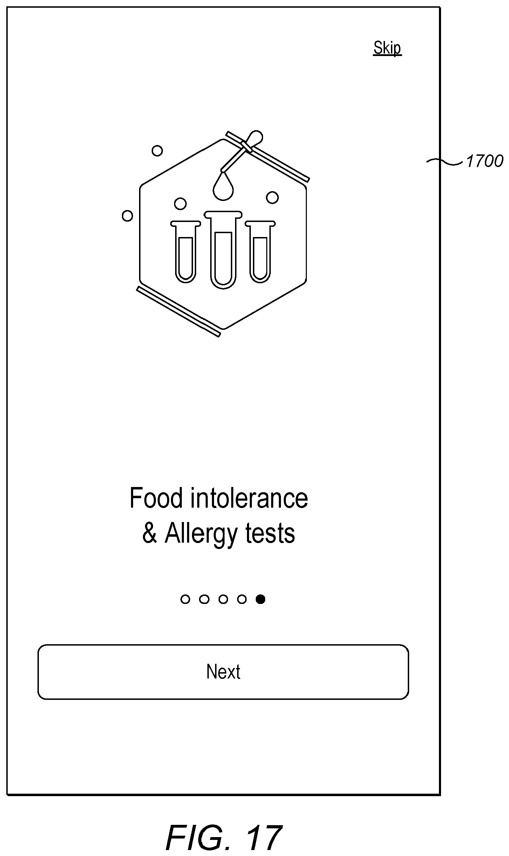

Figure 17 for example shows an initial start screen 1700 of a food intolerance and allergy test.

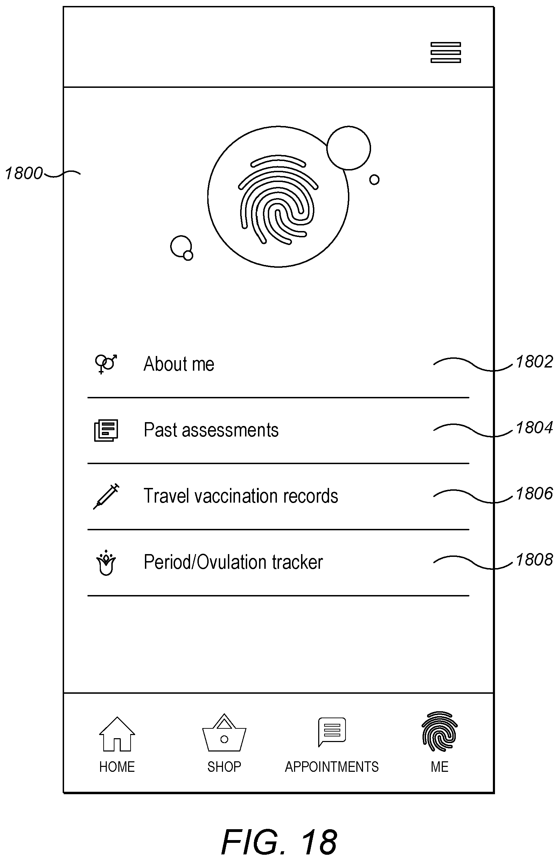

Figure 18 shows a screen 1800 of CAIDR ME. Some example options include "About me" 1802 (e.g. which enables a user to enter their details, which may include biometric details); "Past assessments" 1804 (e.g. where a user can call-up results from earlier assessments); "Travel vaccination records" 1806 (e.g. where a user can obtain up-to-date information of vaccinations they have had); and a "Period/Ovulation tracker" 1808 for female users.

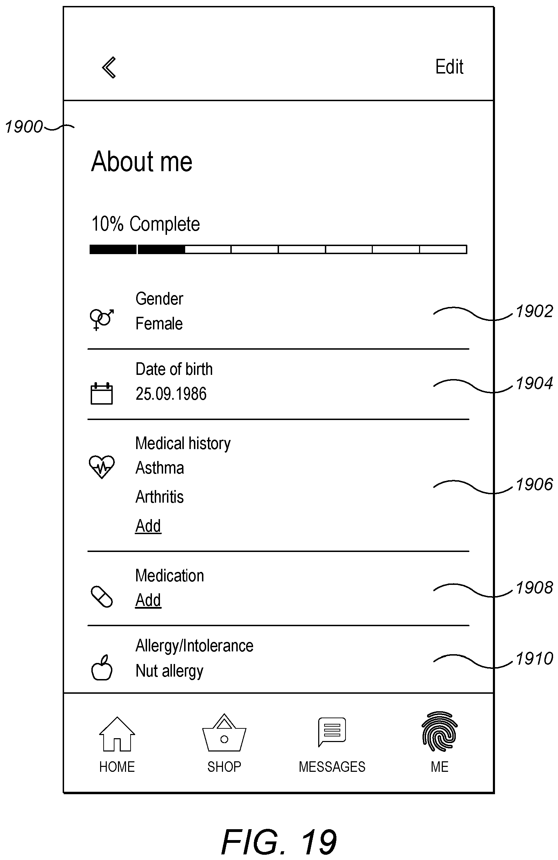

Figure 19 shows an example "About me" screen 1900, which may be displayed after selecting icon 1802 in Fig. 18. This screen shows information of the user e.g. gender 1902; date of birth 1904; medical history 1906; medication 1908; allergy/intolerance information 1910.

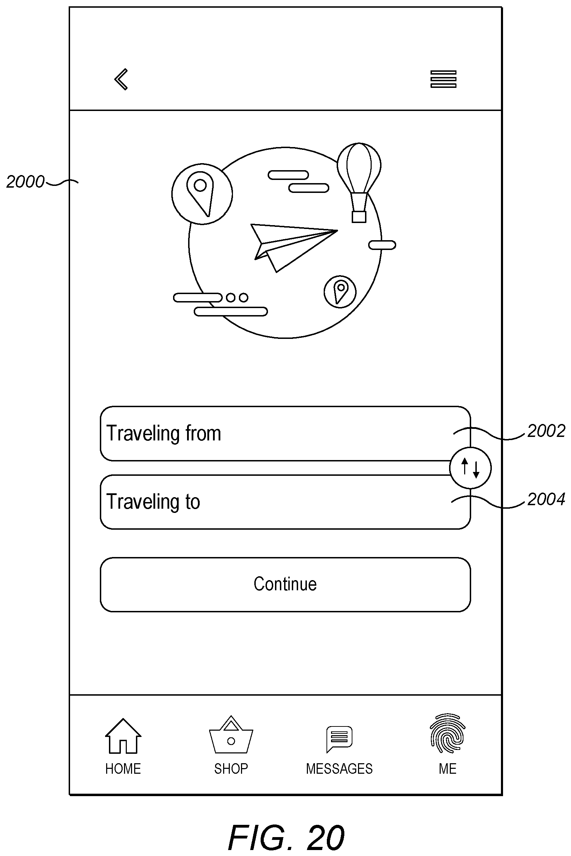

Fig. 20 shows a screen 2000 which may be part of a travel section of the CAIDR app. Via this screen a user can enter information of where they are travelling from 2002 and where they are travelling to 2004. Subsequently the app. can provide recommendations of vaccinations or recommended healthcare products for that trip.

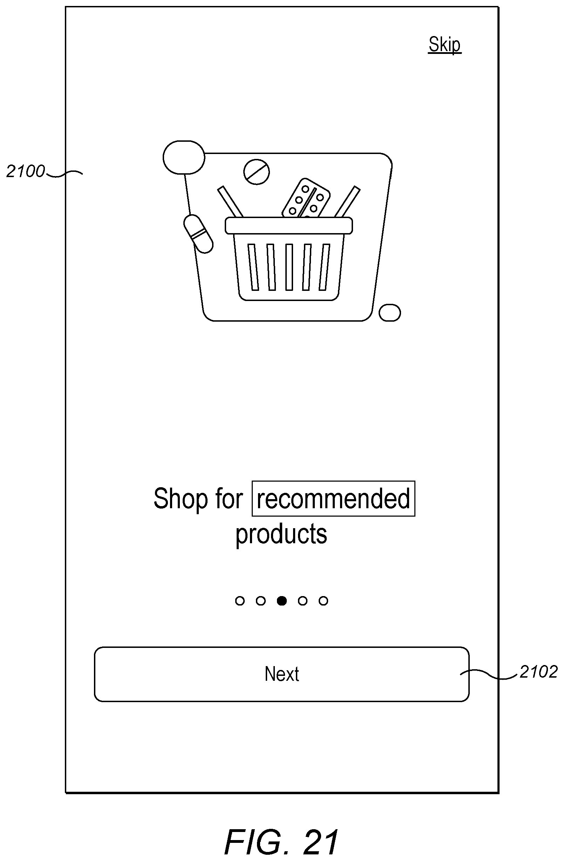

Figure 21 shows a screen 2100, via which a user can purchase one or more products. For example screen 2100 may follow-on from selection of market place 504 icon on the screen shown in Fig. 5.

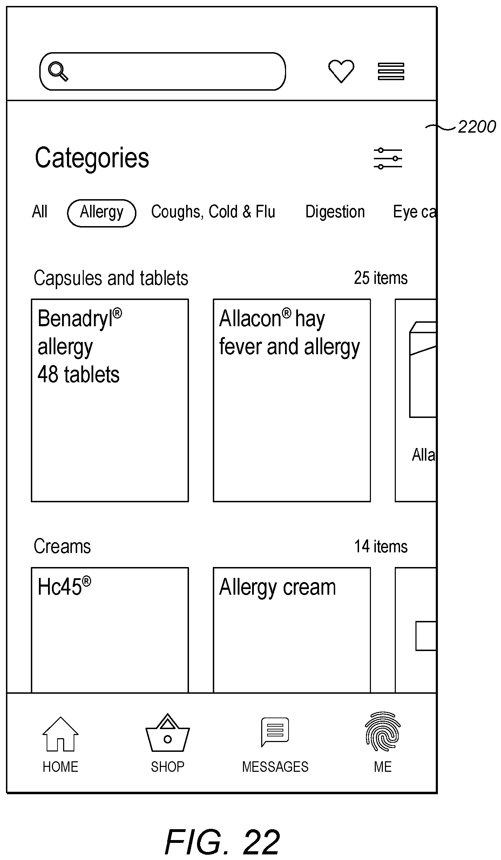

When a user clicks on "Next" icon 2102, the user may be taken to a screen such as screen 2200 shown in Fig. 22, which lists recommended products for purchase.

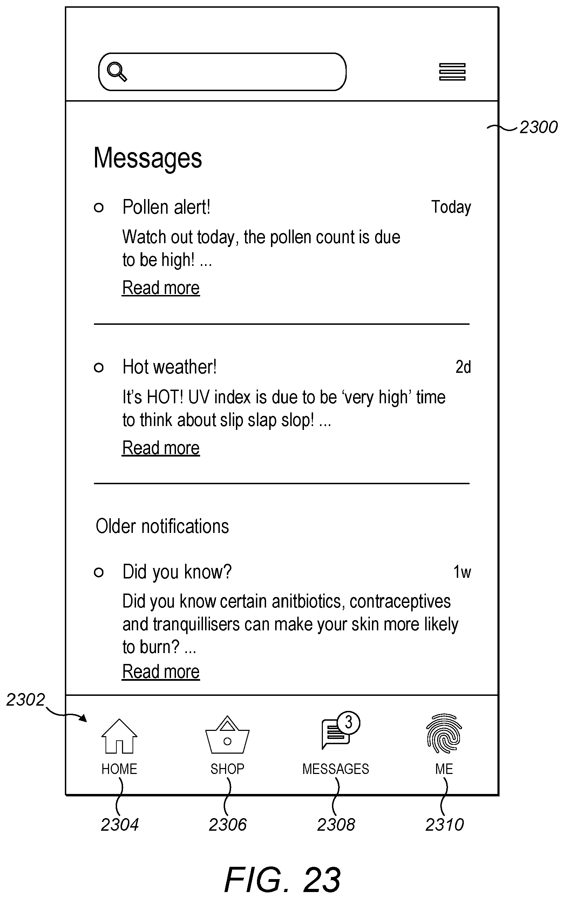

Figure 23 shows a screen 2300 which shows useful messages to the user. In this example the messages are: "Pollen alert!"; "Hot weather!"; as well as a section for older notifications.

Section 2302 of the screen will be noted. Section 2302 comprises a "Home" shortcut icon 2304 (e.g. which will take a user to a home screen such as that shown in Figure 4 or Figure 5); a "Shop" shortcut icon 2306 (e.g. which will take a user to a shop or market-place such as that shown in Figure 21); a "Messages" shortcut icon 2308 (e.g. which will take a user to a messages screen such as that shown in Figure 23); a ME shortcut icon 2310 (e.g. which will take a user to the "ME" section such as that shown in Figure 18). In some examples these shortcut icons are always present on the CAIDR screens (save perhaps for any splash screens such as that shown in Figure 4).

In some examples, users may subscribe to a premium service. Such users may receive a physical "wellness pack" or "wellness box" containing useful healthcare items. The items may include products such as, for example, ORS^{™}, WAYK^{™}, Magastic^{™}, Repelsect^{™}. The wellness pack may be delivered annually, or on a more regular basis. In some examples, a user can choose how often they would like to receive the wellness pack. The wellness pack may also include a drinks bottle, encouraging users to remain hydrated.

Figure 24 shows in more detail a machine 2406 according to an example. The machine 2406 may be equivalent to machine 106 described with respect to Figure 1. In some examples the machine 2406 may be considered a dispenser or vending machine. In some examples the machine 2406 may be considered an intelligent machine dispenser (IMD). In the example of Figure 24, and within the healthcare ecosystem of Figure 1, the machine may be considered a CAIDR NOW machine. The machine 2406 comprises an internal storage area 2402 for storing one or more products to be dispensed. Dispensed products can be provided or dispensed via outlet 2404 of the machine 2406. A display is shown at 2407. In some examples the display comprises a touch-screen. In examples the display is configured to display a user interface (UI) 2408. The user interface 2408 is configured to enable a user to input information which causes the machine 2406 to dispense a product to the user. For example the user interface may enable a user to select a product to be dispensed.

In the example of Figure 24 the actual physical products that are held in storage area 2402 are not visible until dispensed through outlet 2404. In some examples a window is provided on a front face of the dispenser, so as to enable a user to see the products that are contained within the machine 2406.

A card-insertion slot (e.g. debit or credit card) is shown at 2410. A user-interface 2412 is provided that enables a user to enter card details such as a **PIN** number. In some examples a user can pay for a purchased product via card-reader 2410 and user-interface 2412 of the card reader. In some other examples the card-reader 2410 can be used to identify or verify a user who has pre-paid for a product, and in such examples the user interface 2412 may be used to enable a reference number or the like to be entered. Contactless payment via a card is also available to complete a transaction.

The machine 2406 comprises a base 2414, a top 2416, a first side 2418, a second side 2420, a rear side 2423 and a front side 2324. In the example of Figure 24, the front side 2424 comprises a recessed portion 2426. The outlet 2404 is comprised in the recessed portion 2426. Thus it may be considered that the outlet 2404 is comprised in a recessed portion 2426 of a front side of the dispenser 2406. The recessed portion 2426 is positioned above the base 2414 and below the display 2407. According to some examples it is considered that the recessed portion 2426 extends to the base 2414. According to some examples it is considered that the recessed portion 2426 extends between the base 2426 and a position proximate to a lower edge of display 2407. According to some examples it is considered that the recessed portion 2426 extends between the base 2426 and a lower edge of display 2407. The recessed portion 2426 enables wheelchair users to get closer to the display 2407 for interacting with the machine 2406 (e.g. user interface 2408), and for retrieving products from the outlet 2404.

According to some examples the machine 2406 is arranged to store a plurality of products. According to some examples the products comprise consumer healthcare products including but not limited to: aspirin; paracetamol; cough medicine; indigestion remedies, antihistamine medication, Deep Heat ^{®} cream, Deep Freeze ^{®} gel, O.R.S^{™} Hydration Tablets, Canesten ^{®} sachets, Hypromellose ^{®} 0.3% eye drops, Blephagel ^{®}, Care+^{®} olive oil ear drops, Dioralyte ^{®} relief sachets, Fybogel ^{®} Hi Fibre, Repelsect ^{®} patches, Zovirax ^{®} cold sore cream, and Cetraben ^{®} cream.

The machine 2406 also comprises a suitable power source and/or power connector for powering the machine 2406.

In some examples the machine 2406 is configured to receive responses from a user via user interface 2408 e.g. touch input where the display 2407 comprises a touchscreen display. In some examples the machine 2406 comprises a speaker and microphone arrangement, with the speaker configured to generate audible input prompts, and the microphone configured to receive audible responses from the user. In such cases the controller 2431 may comprise voice recognition software in order to recognise the responses.

The machine 2406 comprises a controller shown schematically at 2431. In some examples the controller 2431 comprises a memory 2430 and a processor 2432. A structure of the memory is shown in more detail with respect to Figure 25.

Figure 25 schematically shows a structure of the memory 2430. The memory 2430 stores information of medical symptoms and information of medical conditions, and associations therebetween. The memory 2430 also stores information of products that may be provided to a user to treat their symptoms. In some examples the memory 2430 also stores information of input prompts designed to elicit responses from the user.

As shown schematically in the embodiment of Figure 25 the memory stores a plurality of databases. A first database 2514 stores information of medical symptoms. Such medical symptoms may include symptoms such as cough, dehydration, nausea etc.

A second database 2516 comprises a database of medical conditions. Such medical conditions may comprise for example conjunctivitis, common cold etc.

The memory 2430 further comprises a database of products 2518. This may include stored advice such as advising the user to make an appointment with their GP, to self-medicate, and/or advice of medications or products to take etc.

The memory further comprises a database of input prompts 2520. These prompts may comprise for example one or more questions, or lists of selectable options that a user can select in order to supply information to the controller of their symptoms. The database of input prompts may also be used in order to determine a next input prompt to be displayed (or an output such as a product recommendation), in response to a received input from a user. One or more algorithms may be used in order to control the use and display of input prompts based on user input.

One or more of the databases are associated with each other. This enables for example one or more medical symptoms to be associated with one or more medical conditions. One or more medical symptoms and/or one or more medical conditions may be associated with one or more products. One or more medical symptoms and/or one or more medical conditions may be associated with one or more input prompts.

The aspects of the memory 2430 described with respect to Figure 25 may be considered to comprise a diagnostics engine 2531. The diagnostics engine 2531 is configured to receive information of one or more symptoms, and from that information determine one or more suitable products for treating those symptoms.

For the purposes of explanation an example use case will now be considered with respect to Figure 26.

At S1, a user approaches the machine 2406. In this example the user is experiencing one or more medical symptoms. The user is confronted with a home screen of the user interface 2408. The home screen may contain one or more selectable options, which may include one or more of: (i) search available products; (ii) collect pre-ordered product; (iii) ask dispenser to recommend a product. For (ii), the user may for example enter a code or character string via a user interface or display an identifier to a reader (e.g. a barcode reader) of the machine 2406, which causes a pre-ordered product to be dispensed from outlet 2404.

In some examples the user may be unsure of the best product to treat their medical symptoms. Accordingly the user chooses to ask or request the machine 2406 to recommend a product. In some examples the user will be asked a series of questions via user interface 2408 designed to elicit answers from the user. For example the user may initially indicate that they have a cough, and the user interface 2408 may then display a series of questions to elicit more details about the cough (e.g. whether it is dry or tickly etc.) in order to best recommend a product. That is at S2 it may be considered that the controller 2431 is configured to receive an input from a user via the user interface 2408, the input comprising an indication of one or more medical symptoms of the user.

At S3, the controller 2431 processes the received indication of one or more medical symptoms of the user, to determine a product suitable for helping with the one or more medical symptoms. In examples the controller may use the diagnostics engine 2531 described above for this purpose.

The structures, algorithms and techniques used for receiving inputs from users, generating input prompts, determining a medical condition of a user, and recommending a medical treatment and/or product to the user are described in more detail in GB2561156A and WO2018/171919A1.

**In** response to determining a suitable product, at S4 the controller 2431 causes the machine 2406 to generate a suitable output.

According to some examples, the output comprises dispensing a suitable product. For example, the controller 2431 may have determined from the user input that the user has a headache that is best treated by paracetamol. Accordingly paracetamol may be dispensed. Likewise the controller 2431 may have determined from the user input that the user has blepharitis which is best treated by eye drops. Accordingly eye drops may be dispensed. **In** some examples, there are intermediate steps between the controller determining the product for treatment of the medical condition and the product being dispensed. For example the user may be presented on the user interface 2408 with an indication of the selected product, and an option of whether or not to go-ahead with purchase of the selected product. **In** some cases there may be a plurality of products that have been determined to be suitable. **In** such a case the user may be presented with an indication of each suitable product on the user interface 2408, and the user can make a selection of one or more of the products to select. **In** some examples the controller 2431 determines that there are a number of suitable products, but that some are more suitable than others. **In** such a case the products may be ranked according to suitability, and the user interface 2408 may display the ranking in order to assist the user in making their selection.

In some examples, the controller 2431 is configured to carry out stock-level checks. In some examples the controller 2431 carries out a stock-level check between S3 and S4. The output provided at S4 may therefore differ dependent on the stock-level check. For example, the determination at S3 may be that product "A" is most suitable for the user, but that product "B" is also suitable but less suitable than product A. The stock-level check may reveal that product A is unavailable at the machine 2406. In such an example the output at S4 may present product B to the user as most suitable. In another example the output at S4 may inform the user that product A is most suitable but unavailable at machine 2406, but that product B is available as a suitable alternative. That is in some examples the output comprises information that a most suitable product is not available from the machine 2406 but another suitable product is available from the machine 2406, and providing a user on the user interface 2408 with an option of whether to dispense the another suitable product from the machine 2406.

In some examples the output at S4 comprises information displayed on the user interface 2408 informing a user of where a suitable product can be obtained. For example this may occur when it is determined by the controller 2431 that a suitable product or a most suitable product is not available at machine 2406. For example, information of where a suitable product can be obtained comprises location information. The location information may comprise a map and/or address information. In some examples the location information comprises a location of another dispenser. In some examples the location information comprises information of a vendor, such as a pharmacy. In some examples the machine 2406 is in communication with an external server shown schematically at 2440 (Figure 24), in order to obtain information of where the suitable product is available from. In some examples the controller 2431 takes in to account availability information of the other locations from where the suitable product is available from. For example stock-levels at the other locations may be taken in to account. In some examples, opening time information is taken in to account. For example, the controller may take in to account opening time information of a pharmacy before recommending the user to visit the pharmacy. For example, if the pharmacy closes at 5p.m. and the request for a product is made by the user at machine 2406 at 6p.m., then the controller 2431 may determine not to recommend that pharmacy as a location to obtain the product, or the controller 2431 may cause information to be presented to the user informing the user of the opening times of the pharmacy.

According to some examples, stock-level information of the machine 2406 is made available to one or more third parties. For example the stock-level information may be periodically transmitted by machine 2406 via the external server 2440. Additionally or alternatively, the one or more third parties may be able to monitor stock levels of the machine 2406 by polling the machine 2406 for stock-level information. The stock level information may be polled via server 2440. Accordingly suppliers may be able to understand when stock of a particular product is running low, so that stocks can be replenished.

According to some examples the controller is configured to process cryptocurrency payments, or payments with a proprietary currency such as CAIDR COIN (which may also be referred to as a token). For example a user may send a payment from a device (such as their smartphone) to the dispenser machine 2406, in order to effect payment and obtain a product. This payment may be effected via external server 2440. According to some examples the currency (e.g. CAIDR COIN) is such that it can only be redeemed at certain outlets and/or machines, e.g. at dispenser 2406. This enables an authority, such as a government local authority, to issue funds to persons specifically for the purpose of purchasing healthcare products, safe in the knowledge that those funds cannot be used for other types of purchases.

Figure 27 schematically shows a system 2700 for dispensing one or more healthcare products. A user is schematically shown at 2750. The user 2750 can connect to and communicate with a server 2754 using an electronic device 2752 of the user. The electronic device 2752 may for example comprise a mobile phone or tablet (which may collectively be termed user equipment). The server 2754 may be comprised in the Internet, for example. Either or both of the device 2752 and the server 2754 may comprise the functionality for receiving indications of one or more medical symptoms of a user, to determine a medical condition associated with the indication of one or more medical symptoms so as to recommend a product for treating the medical condition, as described in GB2761156A and WO2018/171919A1.

Therefore it may be considered that the server 2754 is arranged to receive information from a user device 2752 of a user, and to use the received information to process a request for a product to be made available to the user. In some examples the information received from the user device 2752 comprises information of a product e.g. indicating to the server 2754 that the user 2750 requires paracetamol. Or the user device 2752 may forward information of medical symptoms of the user to the server 2754, and the server 2754 processes that information in order to determine a product to be provided to the user. That is one or both of server 2754 and user device 2752 may comprise a diagnostics engine as described above.

One or more apparatus are in communication with server 2754. In some examples two or more apparatus are in communication with server 2754. In the example of Figure 27 a drone 2756, a machine 2758 (which may be equivalent to machine 2206 of Figure 22), and a robot 2760 are in communication with server 2754. Drone 2756, machine 2758 and robot 2760 may individually or collectively be referred to as product-providing apparatus.

In some examples the drone 2756 is located at a repository or storage place of healthcare products. In other examples a home location of drone 2756 may be remote from the repository of healthcare products, the drone being configured to collect one or more healthcare products from the repository on demand. In at least some examples the drone comprises an aerial drone. In some examples the repository of healthcare products comprises a drone.

In some examples the machine 2758 comprises a machine such as machine 2406 described with respect to Figure 24. In other examples the machine 2758 may comprise less functionality than machine 2406. That is in some examples the machine 2758 may not have all the functionality associated with recommending a product to a user (since that functionality may instead be comprised in user device 2752 or server 2754).

The robot 2760 may be located at a pharmacy or other repository of healthcare products. The robot 2760 is configured to fetch or collect an ordered healthcare product and to provide the product to the user. For example when a user enters a pharmacy, the robot 2760 may fetch a product for the user from a shelf or stock-room of the pharmacy. In some examples the robot 2760 comprises a microphone and speaker arrangement for conversing with a user. The robot may comprise voice recognition software for processing speech received from a user.

In some examples the drone 2756 is located at a first location (or configured to obtain a selected product from a first location), the dispenser 2758 is located at a second location, and the robot 2760 is located at a third location. The first location is different from the second location, and the second location is different from the third location (and consequently the first location is different from the third location). In some examples the user 2750 is located at a fourth location, the fourth location being different from the first, second, and third locations.

In Figure 27 and for the purposes of explanation there is shown one user 2750, one drone 2756, one dispenser 2758 and one robot 2760. It will be understood that this is for the purposes of example, and that in reality there may be more than one of each.

According to some examples the server 2754 is arranged to determine at least one apparatus to make a selected product available to a user. For example the server 2754 is arranged to determine which of drone 2756, dispenser 2758 and robot 2760 is most suitable to make a selected product available to a user. For example should it have been determined that the best product for the user is a heartburn-relief product such as Gaviscon^{®}, then the server 2754 determines which of drone 2756, dispenser 2758 and robot 2760 is to provide the Gaviscon^{®} product to the user 2750.

According to some examples, the server is arranged to determine the at least one of the two or more apparatus to make the product available to the user based at least in part on location information. According to some examples the location information comprises a location of the user 2750 (for example based on location of user device 2752). According to some examples the location information comprises location information of the apparatus 2756, 2758, 2760 for providing or dispensing the product. For example the location information may comprise location information of one or more of drone 2756, machine 2758, and robot 2760. According to some examples the location information comprises GPS information such as GPS coordinates.

According to some examples, the determining at least one apparatus to make the product available comprises selecting an apparatus to make the product available.

According to one example the server 2754 is arranged to select the apparatus that is geographically closest to the user 2750. For example if the machine 2758 is closer to the user 2750 than the drone 2756 and the robot 2760, then the server 2754 will determine that the machine 2758 is the preferred apparatus to provide the product to the user 2750. In some examples which apparatus is closest may be determined by straight line distances. In other examples the distance may take in to account actual distance that needs to be travelled e.g. along one or more roads.

According to one example the server is arranged to select the apparatus that can most quickly be reached by the user 2750. For example, the machine 2758 may be closer to the user 2750 than the robot 2760, but the robot may be reached more quickly.

According to one example, the server is arranged to select the apparatus based on information of an ability of a user to travel.

In one example, the information of an ability of a user to travel comprises mobility information of the user. For example, if the user is unable to travel due to incapacity, then the server 2754 may determine that the selected product should be made available to the user 2750 by delivery by drone 2756. In some examples the mobility information of a user is provided by the user. For example this information may have been pre-provided by the user, for example by uploading the information to user device 2752 and/or server 2754. In some examples the mobility information of a user may comprise information of modes of transport that a user is able to take. For example the mobility information of a user may comprise information that user 2750 is able to drive. Therefore, in an example where the machine 2758 is closer to user 2750 than robot 2760, but the location of the robot 2760 is accessible by car and the location of the dispenser 2758 is not accessible by car (e.g. is only accessible by foot), then the server 2754 may select robot 2760 to provide the product rather than machine 2758. Not only does this enhance user convenience, it also may prevent wasted resources (such as fuel and electricity) which may otherwise have been wasted by selecting an apparatus to provide the product that is in fact not accessible by the user 2750 because of the user's mobility constraints.

It will be understood that in some examples the user 2750 may be located in an area which is relatively undeveloped, such as a rural or remote area. For example the user 2750 may be located in an area which is remote from large towns or cities, and/or which may have poor transport infrastructure, and/or which may be prone to adverse weather and/or geological conditions.

Accordingly, in some examples the information of an ability of a user to travel may comprise terrain or mapping information. This may take in to account, for example, terrain which may cause difficulties for a user to reach a product providing apparatus 2756, 2758, 2760. For example there may be a situation where robot 2760 is closer to user 2750 than machine 2758, but there is difficult terrain between user 2750 and robot 2760 (e.g. mountains, a river, a canyon etc.). Accordingly the server 2754 may instead select dispenser 2758 to provide the product, or delivery of the product by drone 2756.

According to some examples the information of an ability of a user to travel may comprise geological information. For example, ordinarily user 2750 may be easily able to travel to machine 2758or robot 2760. However, if for example there has been an earthquake or another geological condition then such travel may not be possible, and instead delivery by drone 2756 may be recommended.

According to some examples the information of an ability of a user to travel may comprise weather information. For example there may be adverse weather conditions in an area between user 2750 and robot 2760, but no adverse weather conditions between user 2750 and machine 2758. Accordingly even if the robot 2760 would under other conditions have been selected to provide the product, under such weather conditions the machine 2758 may instead be selected.

According to some examples the information of an ability of a user to travel may comprise transportation infrastructure information. The transportation infrastructure information may for example take in to account roads, rail, buses, public transport availability, linking the user 2750 to one or more of the product providing apparatus 2756, 2758, 2760. For example the user 2750 may be relatively close to machine 2758, but if located in a rural or remote area then there may be no transport infrastructure linking the user 2750 to the machine 2758 (or indeed to robot 2760). Accordingly in such an example the server 2754 may select delivery of the product by drone 2756.

According to examples the server 2754 is configured to cause an indication to be provided to user 2750 (e.g. via their user device 2752) of the selected apparatus 2756, 2758, 2760 to provide the product to the user. For example the user 2750 may be informed that the product will be delivered by drone 2756. Alternatively the user 2750 may be informed that the product is to be made available from machine 2758 or robot 2760, and may also provide the location of the respective machine 2758or robot 2760. Information such as directions or address information may also be provided to the user to help the user locate the machine 2758 or robot 2760.

According to one example the server 2754 comprises a cloud-server. For example the server 2754 may be physically remote from the user device 2752 and product providing apparatus 2756, 2758, 2760. In another example the server 2754, or at least the functionality of the server 2754, may be provided in one of user device 2752, drone 2756, machine 2758, robot 2760. In some examples the functionality of the server may be made available to user 2750 on their user device 2752 in the form of an application or "app".

In some examples the products to be provided by the product providing apparatus 2756, 2758, 2760 comprise consumer healthcare products. The consumer healthcare products may comprise (by way of non-limiting example), one or more of: paracetamol; aspirin; ibuprofen; decongestant; cough treatment; contraceptive; rehydration treatment; eye-drops; etc.

Figure 28 shows an example apparatus 2852 which may be a user equipment (such as a mobile device or tablet or other electronic device). Apparatus 2852 may for example be analogous to electronic device 2752 of Figure 27, and be able to operate in a system of the type shown in Figure 27.

The apparatus 2852 comprises a memory 2802 and a processor 2804. The apparatus also comprises a display 2806 for displaying information to a user of the apparatus 2852. The apparatus 2852 further comprises input or input means 2808 via which a user can input information into the apparatus 2852. The input means 2808 may comprise for example a keypad comprising one or more symbols such as numbers and letters. Where the display 2806 is a touch screen display, then the input means 2808 may comprise one or more portions of the touchscreen. The apparatus 2852 further comprises means 2810 for communication. The means for communication 2810 may comprise a transmitter and receiver. Via the communication means 2810 the apparatus can communicate wirelessly with one or more external apparatus such as one or more base stations, external servers, the internet etc. The external network is shown generally at 2812. In this example the apparatus 2852 comprises a speaker 2821. The speaker 2821 enables sound or audio to be output from the apparatus 2852. In this example the apparatus 2852 comprises a camera 2823. The camera 2823 enables images to be captured by the apparatus 2852. In this example the camera 2823 can capture still and/or motion picture images. Captured images may be stored in memory 2802. In this example the apparatus 2852 comprises a microphone 2825. The microphone 2825 enables sound or audio to be captured by the apparatus 2852. Captured sounds may be stored in memory 2802.

Apparatus 2852 is configured to store and run a digital healthcare app such as the CAIDR^{®} app. Aspects of operation of such a digital healthcare app are described in GB2561156A and WO2018/171919A1. In summary, the app is configured to determine a medical condition associated with an indication of one or more medical symptoms provided by a user, and then provides an output in the form of advice to the user of how to respond to the medical condition (such as a recommendation of a product to treat the medical condition).

It will be understood that the processor or processing system or circuitry referred to herein may in practice be provided by a single chip or integrated circuit or plural chips or integrated circuits, optionally provided as a chipset, an application-specific integrated circuit (ASIC), field-programmable gate array (FPGA), digital signal processor (DSP), graphics processing units (GPUs), etc. The chip or chips may comprise circuitry (as well as possibly firmware) for embodying at least one or more of a data processor or processors, a digital signal processor or processors, baseband circuitry and radio frequency circuitry, which are configurable so as to operate in accordance with the exemplary embodiments. In this regard, the exemplary embodiments may be implemented at least in part by computer software stored in (non-transitory) memory and executable by the processor, or by hardware, or by a combination of tangibly stored software and hardware (and tangibly stored firmware).

Reference is made herein to data storage for storing data. This may be provided by a single device or by plural devices. Suitable devices include for example a hard disk and non-volatile semiconductor memory.

The examples described herein are to be understood as illustrative examples of embodiments of the invention. Further embodiments and examples are envisaged. Any feature described in relation to any one example or embodiment may be used alone or in combination with other features. In addition, any feature described in relation to any one example or embodiment may also be used in combination with one or more features of any other of the examples or embodiments, or any combination of any other of the examples or embodiments. For example, aspects from the system of Figure 27 may be combined with aspects of the systems of Figures 1 to 3. Furthermore, modifications may also be employed within the scope of the invention, which is defined in the claims.

## Claims

1. A system comprising:
a server (2754) arranged to receive information from a user device (2752) of a user (2750), and to use the received information to process a request for a product to be made available to the user (2750);
two or more apparatus in communication with the server (2754);
the server (2754) arranged to determine at least one of the two or more apparatus to make the product available to the user (2750), and to provide an indication to the user (2750) of a selection of the at least one of the two or more apparatus, the server (2754) arranged to determine the at least one of the two or more apparatus to make the product available to the user (2750) based at least in part on location information, the location information comprising location information of the user (2750) and location information of the two or more apparatus;
**characterised in that** the server (2754) is arranged to select at least one of the two or more apparatus based on information of an ability of the user (2750) to travel, wherein the information of an ability to travel is based upon one or more of: terrain information; or geological information;
at least one of the two or more apparatus comprises a drone (2756) arranged to deliver the product to the user (2750), wherein the server (2754) is arranged to select the drone (2756) to deliver the product to the user (2750) when travel of the user (2750) to another of the two or more apparatus is determined as difficult or not possible, based on the terrain information and/or the geological information; and
wherein the product comprises a consumer healthcare product selected from a plurality of available consumer healthcare products using a diagnostics engine (2531) for determining one or more medical symptoms of the user (2750), wherein the diagnostics engine (2531) is comprised in one or more of: the server (2754); the user device (2752) of the user (2750).

2. A system according to claim 1, wherein the drone (2756) is arranged to deliver the product to the user (2750) from a repository of products and preferably wherein the repository of products comprises a pharmacy.

3. A system according to any of claims 1 to 2, at least one of the two or more apparatus comprising a machine (2758) arranged to dispense the product to the user (2750) and preferably wherein the machine (2758) comprises a vending machine.

4. A system according to any of claims 1 to 3, at least one of the two or more apparatus comprising a robot (2760) arranged to provide the product for the user (2750).

5. A system according to any of claims 1 to 4, comprising a payment system (120).

6. A system according to claim 5, wherein the payment system (120) utilises a proprietary currency and preferably wherein the payment system (120) enables an authority to track payments made via the payment system (120).

7. A system according to any of claims 1 to 6, comprising an interactive tool enabling a user (2750) to perform one or more actions.

8. A system according to claim 7, the one or more actions comprising: a health assessment; a drug interaction test to determine whether there are any drugs that the user (2750) is allergic to; a sports test; tracking menstrual cycle; tracking weight; tracking sleep pattern; providing one or more bio-markers for assessment.

9. A system according to claim 7 or claim 8, the interactive tool configured to utilise a facial recognition algorithm to determine conditions of one or more of: skin condition; depression; stress; high/low blood pressure of a user (2750).

10. A system according to any of claims 1 to 9, comprising a research and development engine (122) which is configured to, over time, learn efficacy of one or more products by one or more of: sales data; user reviews; surveys.

11. A system according to any of claims 1 to 10, configured to enable a live video chat between a user (2750) and a pharmacist (348).

12. A server (2754) comprising:
a controller (2431) arranged to receive information from a user device (2752) of a user (2750), and to use the received information to process a request for a product to be made available to the user (2750);
the controller (2431) arranged to determine at least one of two or more apparatus to make the product available to the user (2750), and to provide an indication to the user (2750) of a selection of the at least one of the two or more apparatus, the controller (2431) arranged to determine the at least one of the two or more apparatus to make the product available to the user (2750) based at least in part on location information, the location information comprising location information of the user (2750) and location information of the two or more apparatus;
**characterised in that** the controller (2431) is arranged to select at least one of the two or more apparatus based on information of an ability of the user (2750) to travel, wherein the information of an ability to travel is based upon one or more of: terrain information; or geological information;
at least one of the two or more apparatus comprises a drone (2756) arranged to deliver the product to the user (2750), wherein the controller (2431) is arranged to select the drone (2756) to deliver the product to the user (2750) when travel of the user (2750) to another of the two or more apparatus is determined as difficult or not possible, based on the terrain information and/or the geological information; and
wherein the product comprises a consumer healthcare product selected from a plurality of available consumer healthcare products using a diagnostics engine (2531) for determining one or more medical symptoms of the user (2750), wherein the diagnostics engine (2531) is comprised in one or more of: the server (2754); the user device (2752) of the user (2750).

13. A method comprising:
receiving information from a user device (2752) of a user (2750);
using the received information to process a request for a product to be made available to the user (2750);
determining at least one of two or more apparatus to make the product available to the user (2750);
the determining the at least one of the two or more apparatus to make the product available to the user (2750) based at least in part on location information, the location information comprising location information of the user (2750) and location information of the two or more apparatus;
providing an indication to the user (2750) of a selection of the at least one of the two or more apparatus;
**characterised by** selecting at least one of the two or more apparatus based on information of an ability of the user (2750) to travel, wherein the information of an ability to travel is based upon one or more of: terrain information; or geological information;
wherein at least one of the two or more apparatus comprises a drone (2756) arranged to deliver the product to the user (2750) and selecting the drone (2756) to deliver the product to the user (2750) when travel of the user (2750) to another of the two or more apparatus is determined as difficult or not possible, based on the terrain information and/or the geological information; and
wherein the product comprises a consumer healthcare product and selecting the product from a plurality of available consumer healthcare products using a diagnostics engine (2531) for determining one or more medical symptoms of the user (2750), wherein the diagnostics engine (2531) is comprised in one or more of: a server (2754); the user device (2752) of the user (2750).

14. A computer program comprising computer executable instructions which when run on one or more processors perform:
receiving information from a user device (2752) of a user (2750);
using the received information to process a request for a product to be made available to the user (2750);
determining at least one of two or more apparatus to make the product available to the user (2750);
the determining the at least one of the two or more apparatus to make the product available to the user (2750) based at least in part on location information, the location information comprising location information of the user (2750) and location information of the two or more apparatus;
providing an indication to the user (2750) of a selection of the at least one of the two or more apparatus;
**characterised by** selecting at least one of the two or more apparatus based on information of an ability of the user (2750) to travel, wherein the information of an ability to travel is based upon one or more of: terrain information; or geological information;
wherein at least one of the two or more apparatus comprises a drone (2756) arranged to deliver the product to the user (2750) and selecting the drone (2756) to deliver the product to the user (2750) when travel of the user (2750) to another of the two or more apparatus is determined as difficult or not possible, based on the terrain information and/or the geological information; and
wherein the product comprises a consumer healthcare product and selecting the product from a plurality of available consumer healthcare products using a diagnostics engine (2531) for determining one or more medical symptoms of the user (2750), wherein the diagnostics engine (2531) is comprised in one or more of: a server (2754); the user device (2752) of the user (2750).

## Patentansprüche

1. System, umfassend:
einen Server (2754), der dazu eingerichtet ist, Informationen von einer Benutzervorrichtung (2752) eines Benutzers (2750) zu empfangen und die empfangenen Informationen zu verwenden, um eine Anforderung für ein Produkt zu verarbeiten, das dem Benutzer (2750) zur Verfügung gestellt werden soll;
zwei oder mehr Vorrichtungen, die mit dem Server (2754) in Kommunikation stehen;
wobei der Server (2754) dazu eingerichtet ist, mindestens eine der zwei oder mehr Vorrichtungen zu bestimmen, um das Produkt dem Benutzer (2750) zur Verfügung zu stellen, und dem Benutzer (2750) eine Angabe einer Auswahl der mindestens einen der zwei oder mehr Vorrichtungen bereitzustellen, wobei der Server (2754) dazu eingerichtet ist, die mindestens eine der zwei oder mehr Vorrichtungen zu bestimmen, um das Produkt dem Benutzer (2750) zur Verfügung zu stellen, zumindest teilweise basierend auf Standortinformationen, wobei die Standortinformationen Standortinformationen des Benutzers (2750) und Standortinformationen der zwei oder mehr Vorrichtungen umfassen;
**dadurch gekennzeichnet, dass** der Server (2754) dazu eingerichtet ist, mindestens eine der zwei oder mehr Vorrichtungen basierend auf Informationen über eine Fähigkeit des Benutzers (2750), sich fortzubewegen, auszuwählen, wobei die Informationen über eine Fähigkeit, sich fortzubewegen, auf einem oder mehreren der folgenden Elemente basieren: Geländeinformationen; oder geologischen Informationen;
wobei mindestens eine der zwei oder mehr Vorrichtungen eine Drohne (2756) umfasst, die dazu eingerichtet ist, das Produkt an den Benutzer (2750) zu liefern, wobei der Server (2754) dazu eingerichtet ist, die Drohne (2756) auszuwählen, um das Produkt an den Benutzer (2750) zu liefern, wenn bestimmt wird, dass die Fortbewegung des Benutzers (2750) zu einer anderen der zwei oder mehr Vorrichtungen schwierig oder nicht möglich ist, basierend auf den Geländeinformationen und/oder den geologischen Informationen; und
wobei das Produkt ein Verbraucher-Gesundheitsprodukt umfasst, das aus einer Vielzahl verfügbarer Verbraucher-Gesundheitsprodukte unter Verwendung einer Diagnose-Engine (2531) ausgewählt wird, die dazu dient, ein oder mehrere medizinische Symptome des Benutzers (2750) zu bestimmen, wobei die Diagnose-Engine (2531) in einem oder mehreren der folgenden Elemente enthalten ist: dem Server (2754); der Benutzervorrichtung (2752) des Benutzers (2750).

2. System nach Anspruch 1, wobei die Drohne (2756) dazu eingerichtet ist, das Produkt aus einem Produktdepot an den Benutzer (2750) zu liefern, und vorzugsweise wobei das Produktdepot eine Apotheke umfasst.

3. System nach einem der Ansprüche 1 bis 2, wobei mindestens eine der zwei oder mehr Vorrichtungen eine Maschine (2758) umfasst, die dazu eingerichtet ist, das Produkt an den Benutzer (2750) auszugeben, und vorzugsweise wobei die Maschine (2758) einen Verkaufsautomaten umfasst.

4. System nach einem der Ansprüche 1 bis 3, wobei mindestens eine der zwei oder mehr Vorrichtungen einen Roboter (2760) umfasst, der dazu eingerichtet ist, das Produkt für den Benutzer (2750) bereitzustellen.

5. System nach einem der Ansprüche 1 bis 4, umfassend ein Zahlungssystem (120).

6. System nach Anspruch 5, wobei das Zahlungssystem (120) eine proprietäre Währung verwendet und vorzugsweise wobei das Zahlungssystem (120) einer Behörde ermöglicht, über das Zahlungssystem (120) getätigte Zahlungen zu verfolgen.

7. System nach einem der Ansprüche 1 bis 6, umfassend ein interaktives Werkzeug, das einem Benutzer (2750) ermöglicht, eine oder mehrere Aktionen durchzuführen.

8. System nach Anspruch 7, wobei die eine oder die mehreren Aktionen Folgendes umfassen: eine Gesundheitsbewertung; einen Arzneimittelwechselwirkungstest zum Bestimmen, ob Arzneimittel vorhanden sind, gegen die der Benutzer (2750) allergisch ist; einen Sporttest; das Verfolgen des Menstruationszyklus; das Verfolgen des Gewichts; das Verfolgen des Schlafmusters; das Bereitstellen eines oder mehrerer Biomarker zur Bewertung.

9. System nach Anspruch 7 oder Anspruch 8, wobei das interaktive Werkzeug dazu konfiguriert ist, einen Gesichtserkennungsalgorithmus zu verwenden, um einen oder mehrere der folgenden Zustände eines Benutzers (2750) zu bestimmen: Hauterkrankung; Depression; Stress; hoher/niedriger Blutdruck.

10. System nach einem der Ansprüche 1 bis 9, umfassend eine Forschungs- und Entwicklungs-Engine (122), die dazu konfiguriert ist, im Laufe der Zeit die Wirksamkeit eines oder mehrerer Produkte anhand eines oder mehrerer der folgenden Elemente zu erlernen: Verkaufsdaten; Benutzerbewertungen; Umfragen.

11. System nach einem der Ansprüche 1 bis 10, das dazu konfiguriert ist, einen Live-Video-Chat zwischen einem Benutzer (2750) und einem Apotheker (348) zu ermöglichen.

12. Server (2754), umfassend:
einen Controller (2431), der dazu eingerichtet ist, Informationen von einer Benutzervorrichtung (2752) eines Benutzers (2750) zu empfangen und die empfangenen Informationen zu verwenden, um eine Anforderung für ein Produkt zu verarbeiten, das dem Benutzer (2750) zur Verfügung gestellt werden soll;
wobei der Controller (2431) dazu eingerichtet ist, mindestens eine aus zwei oder mehr Vorrichtungen zu bestimmen, um das Produkt dem Benutzer (2750) zur Verfügung zu stellen, und dem Benutzer (2750) eine Angabe einer Auswahl der mindestens einen der zwei oder mehr Vorrichtungen bereitzustellen, wobei der Controller (2431) dazu eingerichtet ist, die mindestens eine der zwei oder mehr Vorrichtungen zu bestimmen, um das Produkt dem Benutzer (2750) zur Verfügung zu stellen, zumindest teilweise basierend auf Standortinformationen, wobei die Standortinformationen Standortinformationen des Benutzers (2750) und Standortinformationen der zwei oder mehr Vorrichtungen umfassen;
**dadurch gekennzeichnet, dass** der Controller (2431) dazu eingerichtet ist, mindestens eine der zwei oder mehr Vorrichtungen basierend auf Informationen über eine Fähigkeit des Benutzers (2750), sich fortzubewegen, auszuwählen, wobei die Informationen über eine Fähigkeit, sich fortzubewegen, auf einem oder mehreren der folgenden Elemente basieren: Geländeinformationen; oder geologischen Informationen;
wobei mindestens eine der zwei oder mehr Vorrichtungen eine Drohne (2756) umfasst, die dazu eingerichtet ist, das Produkt an den Benutzer (2750) zu liefern, wobei der Controller (2431) dazu eingerichtet ist, die Drohne (2756) auszuwählen, um das Produkt an den Benutzer (2750) zu liefern, wenn bestimmt wird, dass die Fortbewegung des Benutzers (2750) zu einer anderen der zwei oder mehr Vorrichtungen schwierig oder nicht möglich ist, basierend auf den Geländeinformationen und/oder den geologischen Informationen; und
wobei das Produkt ein Verbraucher-Gesundheitsprodukt umfasst, das aus einer Vielzahl verfügbarer Verbraucher-Gesundheitsprodukte unter Verwendung einer Diagnose-Engine (2531) ausgewählt wird, die dazu dient, ein oder mehrere medizinische Symptome des Benutzers (2750) zu bestimmen, wobei die Diagnose-Engine (2531) in einem oder mehreren der folgenden Elemente enthalten ist: dem Server (2754); der Benutzervorrichtung (2752) des Benutzers (2750).

13. Verfahren, umfassend:
Empfangen von Informationen von einer Benutzervorrichtung (2752) eines Benutzers (2750);
Verwenden der empfangenen Informationen, um eine Anforderung für ein Produkt zu verarbeiten, das dem Benutzer (2750) zur Verfügung gestellt werden soll;
Bestimmen mindestens einer aus zwei oder mehr Vorrichtungen, um das Produkt dem Benutzer (2750) zur Verfügung zu stellen;
wobei das Bestimmen der mindestens einen der zwei oder mehr Vorrichtungen, um das Produkt dem Benutzer (2750) zur Verfügung zu stellen, zumindest teilweise auf Standortinformationen basiert, wobei die Standortinformationen Standortinformationen des Benutzers (2750) und Standortinformationen der zwei oder mehr Vorrichtungen umfassen;
Bereitstellen einer Angabe einer Auswahl der mindestens einen der zwei oder mehr Vorrichtungen an den Benutzer (2750);
**dadurch gekennzeichnet, dass** mindestens eine der zwei oder mehr Vorrichtungen basierend auf Informationen über eine Fähigkeit des Benutzers (2750), sich fortzubewegen, ausgewählt wird, wobei die Informationen über eine Fähigkeit, sich fortzubewegen, auf einem oder mehreren der folgenden Elemente basieren: Geländeinformationen; oder geologischen Informationen;
wobei mindestens eine der zwei oder mehr Vorrichtungen eine Drohne (2756) umfasst, die dazu eingerichtet ist, das Produkt an den Benutzer (2750) zu liefern, und die Drohne (2756) ausgewählt wird, um das Produkt an den Benutzer (2750) zu liefern, wenn bestimmt wird, dass die Fortbewegung des Benutzers (2750) zu einer anderen der zwei oder mehr Vorrichtungen schwierig oder nicht möglich ist, basierend auf den Geländeinformationen und/oder den geologischen Informationen; und
wobei das Produkt ein Verbraucher-Gesundheitsprodukt umfasst und das Produkt aus einer Vielzahl verfügbarer Verbraucher-Gesundheitsprodukte unter Verwendung einer Diagnose-Engine (2531) ausgewählt wird, die dazu dient, ein oder mehrere medizinische Symptome des Benutzers (2750) zu bestimmen, wobei die Diagnose-Engine (2531) in einem oder mehreren der folgenden Elemente enthalten ist: einem Server (2754); der Benutzervorrichtung (2752) des Benutzers (2750).

14. Computerprogramm, umfassend computerausführbare Anweisungen, die, wenn sie auf einem oder mehreren Prozessoren ausgeführt werden, Folgendes bewirken:
Empfangen von Informationen von einer Benutzervorrichtung (2752) eines Benutzers (2750);
Verwenden der empfangenen Informationen, um eine Anforderung für ein Produkt zu verarbeiten, das dem Benutzer (2750) zur Verfügung gestellt werden soll;
Bestimmen mindestens einer aus zwei oder mehr Vorrichtungen, um das Produkt dem Benutzer (2750) zur Verfügung zu stellen;
wobei das Bestimmen der mindestens einen der zwei oder mehr Vorrichtungen, um das Produkt dem Benutzer (2750) zur Verfügung zu stellen, zumindest teilweise auf Standortinformationen basiert, wobei die Standortinformationen Standortinformationen des Benutzers (2750) und Standortinformationen der zwei oder mehr Vorrichtungen umfassen;
Bereitstellen einer Angabe einer Auswahl der mindestens einen der zwei oder mehr Vorrichtungen an den Benutzer (2750);
**dadurch gekennzeichnet, dass** mindestens eine der zwei oder mehr Vorrichtungen basierend auf Informationen über eine Fähigkeit des Benutzers (2750), sich fortzubewegen, ausgewählt wird, wobei die Informationen über eine Fähigkeit, sich fortzubewegen, auf einem oder mehreren der folgenden Elemente basieren: Geländeinformationen; oder geologischen Informationen;
wobei mindestens eine der zwei oder mehr Vorrichtungen eine Drohne (2756) umfasst, die dazu eingerichtet ist, das Produkt an den Benutzer (2750) zu liefern, und die Drohne (2756) ausgewählt wird, um das Produkt an den Benutzer (2750) zu liefern, wenn bestimmt wird, dass die Fortbewegung des Benutzers (2750) zu einer anderen der zwei oder mehr Vorrichtungen schwierig oder nicht möglich ist, basierend auf den Geländeinformationen und/oder den geologischen Informationen; und
wobei das Produkt ein Verbraucher-Gesundheitsprodukt umfasst und das Produkt aus einer Vielzahl verfügbarer Verbraucher-Gesundheitsprodukte unter Verwendung einer Diagnose-Engine (2531) ausgewählt wird, die dazu dient, ein oder mehrere medizinische Symptome des Benutzers (2750) zu bestimmen, wobei die Diagnose-Engine (2531) in einem oder mehreren der folgenden Elemente enthalten ist: einem Server (2754); der Benutzervorrichtung (2752) des Benutzers (2750).

## Revendications

1. Système comprenant :
un serveur (2754) agencé pour recevoir des informations provenant d'un dispositif utilisateur (2752) d'un utilisateur (2750), et pour utiliser les informations reçues afin de traiter une demande pour un produit devant être mis à la disposition de l'utilisateur (2750) ;
deux appareils ou plus en communication avec le serveur (2754) ;
le serveur (2754) étant agencé pour déterminer au moins l'un des deux appareils ou plus afin de mettre le produit à la disposition de l'utilisateur (2750), et pour fournir à l'utilisateur (2750) une indication d'une sélection de l'au moins un des deux appareils ou plus, le serveur (2754) étant agencé pour déterminer l'au moins un des deux appareils ou plus afin de mettre le produit à la disposition de l'utilisateur (2750) sur la base, au moins en partie, d'informations de localisation, les informations de localisation comprenant des informations de localisation de l'utilisateur (2750) et des informations de localisation des deux appareils ou plus ;
**caractérisé en ce que** le serveur (2754) est agencé pour sélectionner au moins l'un des deux appareils ou plus sur la base d'informations relatives à une capacité de l'utilisateur (2750) à se déplacer, dans lequel les informations relatives à une capacité à se déplacer sont basées sur l'une ou plusieurs : d'informations relatives au terrain ; ou d'informations géologiques ;
au moins l'un des deux appareils ou plus comprend un drone (2756) agencé pour délivrer le produit à l'utilisateur (2750), dans lequel le serveur (2754) est agencé pour sélectionner le drone (2756) pour délivrer le produit à l'utilisateur (2750) lorsque le déplacement de l'utilisateur (2750) vers un autre des deux appareils ou plus est déterminé comme étant difficile ou impossible, sur la base des informations relatives au terrain et/ou des informations géologiques ; et
dans lequel le produit comprend un produit de santé destiné au consommateur sélectionné parmi une pluralité de produits de santé destinés au consommateur disponibles à l'aide d'un moteur de diagnostic (2531) pour déterminer un ou plusieurs symptômes médicaux de l'utilisateur (2750), dans lequel le moteur de diagnostic (2531) est compris dans l'un ou plusieurs : du serveur (2754) ; du dispositif utilisateur (2752) de l'utilisateur (2750).

2. Système selon la revendication 1, dans lequel le drone (2756) est agencé pour délivrer le produit à l'utilisateur (2750) depuis un dépôt de produits et, de préférence, dans lequel le dépôt de produits comprend une pharmacie.

3. Système selon l'une quelconque des revendications 1 à 2, au moins l'un des deux appareils ou plus comprenant une machine (2758) agencée pour distribuer le produit à l'utilisateur (2750) et, de préférence, dans lequel la machine (2758) comprend un distributeur automatique.

4. Système selon l'une quelconque des revendications 1 à 3, au moins l'un des deux appareils ou plus comprenant un robot (2760) agencé pour fournir le produit à l'utilisateur (2750).

5. Système selon l'une quelconque des revendications 1 à 4, comprenant un système de paiement (120).

6. Système selon la revendication 5, dans lequel le système de paiement (120) utilise une monnaie propriétaire et, de préférence, dans lequel le système de paiement (120) permet à une autorité de suivre les paiements effectués par l'intermédiaire du système de paiement (120).

7. Système selon l'une quelconque des revendications 1 à 6, comprenant un outil interactif permettant à un utilisateur (2750) d'effectuer une ou plusieurs actions.

8. Système selon la revendication 7, les une ou plusieurs actions comprenant : une évaluation de santé ; un test d'interaction médicamenteuse destiné à déterminer s'il existe des médicaments auxquels l'utilisateur (2750) est allergique ; un test sportif ; le suivi du cycle menstruel ; le suivi du poids ; le suivi des habitudes de sommeil ; la fourniture d'un ou de plusieurs biomarqueurs pour évaluation.

9. Système selon la revendication 7 ou la revendication 8, dans lequel l'outil interactif est configuré pour utiliser un algorithme de reconnaissance faciale afin de déterminer des états de l'un ou de plusieurs : d'une pathologie cutanée ; d'une dépression ; d'un stress ; d'une pression artérielle élevée/faible d'un utilisateur (2750).

10. Système selon l'une quelconque des revendications 1 à 9, comprenant un moteur de recherche et développement (122) qui est configuré pour apprendre, au fil du temps, l'efficacité d'un ou de plusieurs produits par l'un ou de plusieurs : de données de vente ; d'avis d'utilisateurs ; d'enquêtes.

11. Système selon l'une quelconque des revendications 1 à 10, configuré pour permettre une conversation vidéo en direct entre un utilisateur (2750) et un pharmacien (348).

12. Serveur (2754) comprenant :
un contrôleur (2431) agencé pour recevoir des informations provenant d'un dispositif utilisateur (2752) d'un utilisateur (2750), et pour utiliser les informations reçues afin de traiter une demande pour un produit devant être mis à la disposition de l'utilisateur (2750) ;
le contrôleur (2431) étant agencé pour déterminer au moins l'un de deux appareils ou plus afin de mettre le produit à la disposition de l'utilisateur (2750), et pour fournir à l'utilisateur (2750) une indication d'une sélection de l'au moins un des deux appareils ou plus, le contrôleur (2431) étant agencé pour déterminer l'au moins un des deux appareils ou plus afin de mettre le produit à la disposition de l'utilisateur (2750) sur la base, au moins en partie, d'informations de localisation, les informations de localisation comprenant des informations de localisation de l'utilisateur (2750) et des informations de localisation des deux appareils ou plus ;
**caractérisé en ce que** le contrôleur (2431) est agencé pour sélectionner au moins l'un des deux appareils ou plus sur la base d'informations relatives à une capacité de l'utilisateur (2750) à se déplacer, dans lequel les informations relatives à une capacité à se déplacer sont basées sur l'une ou plusieurs : d'informations relatives au terrain ; ou d'informations géologiques ;
au moins l'un des deux appareils ou plus comprend un drone (2756) agencé pour délivrer le produit à l'utilisateur (2750), dans lequel le contrôleur (2431) est agencé pour sélectionner le drone (2756) pour délivrer le produit à l'utilisateur (2750) lorsque le déplacement de l'utilisateur (2750) vers un autre des deux appareils ou plus est déterminé comme étant difficile ou impossible, sur la base des informations relatives au terrain et/ou des informations géologiques ; et
dans lequel le produit comprend un produit de santé destiné au consommateur sélectionné parmi une pluralité de produits de santé destinés au consommateur disponibles à l'aide d'un moteur de diagnostic (2531) pour déterminer un ou plusieurs symptômes médicaux de l'utilisateur (2750), dans lequel le moteur de diagnostic (2531) est compris dans l'un ou plusieurs : du serveur (2754) ; du dispositif utilisateur (2752) de l'utilisateur (2750).

13. Procédé comprenant :
la réception d'informations provenant d'un dispositif utilisateur (2752) d'un utilisateur (2750) ;
l'utilisation des informations reçues pour traiter une demande relative à un produit devant être mis à la disposition de l'utilisateur (2750) ;
la détermination d'au moins l'un de deux appareils ou plus afin de mettre le produit à la disposition de l'utilisateur (2750) ;
la détermination de l'au moins un des deux appareils ou plus afin de mettre le produit à la disposition de l'utilisateur (2750) sur la base, au moins en partie, d'informations de localisation, les informations de localisation comprenant des informations de localisation de l'utilisateur (2750) et des informations de localisation des deux appareils ou plus ;
la fourniture à l'utilisateur (2750) d'une indication d'une sélection de l'au moins un des deux appareils ou plus ;
**caractérisé par** la sélection d'au moins l'un des deux appareils ou plus sur la base d'informations relatives à une capacité de l'utilisateur (2750) à se déplacer, dans lequel les informations relatives à une capacité à se déplacer sont basées sur l'une ou plusieurs :
d'informations relatives au terrain ; ou d'informations géologiques ;
dans lequel au moins l'un des deux appareils ou plus comprend un drone (2756) agencé pour délivrer le produit à l'utilisateur (2750) et la sélection du drone (2756) pour délivrer le produit à l'utilisateur (2750) lorsque le déplacement de l'utilisateur (2750) vers un autre des deux appareils ou plus est déterminé comme étant difficile ou impossible, sur la base des informations relatives au terrain et/ou des informations géologiques ; et
dans lequel le produit comprend un produit de santé destiné au consommateur et la sélection du produit parmi une pluralité de produits de santé destinés au consommateur disponibles à l'aide d'un moteur de diagnostic (2531) pour déterminer un ou plusieurs symptômes médicaux de l'utilisateur (2750), dans lequel le moteur de diagnostic (2531) est compris dans l'un ou plusieurs : d'un serveur (2754) ; du dispositif utilisateur (2752) de l'utilisateur (2750).

14. Programme informatique comprenant des instructions exécutables par ordinateur qui, lorsqu'elles sont exécutées sur un ou plusieurs processeurs, exécutent :
la réception d'informations provenant d'un dispositif utilisateur (2752) d'un utilisateur (2750) ;
l'utilisation des informations reçues pour traiter une demande relative à un produit devant être mis à la disposition de l'utilisateur (2750) ;
la détermination d'au moins l'un de deux appareils ou plus afin de mettre le produit à la disposition de l'utilisateur (2750) ;
la détermination de l'au moins un des deux appareils ou plus afin de mettre le produit à la disposition de l'utilisateur (2750) sur la base, au moins en partie, d'informations de localisation, les informations de localisation comprenant des informations de localisation de l'utilisateur (2750) et des informations de localisation des deux appareils ou plus ;
la fourniture à l'utilisateur (2750) d'une indication d'une sélection de l'au moins un des deux appareils ou plus ;
**caractérisé par** la sélection d'au moins l'un des deux appareils ou plus sur la base d'informations relatives à une capacité de l'utilisateur (2750) à se déplacer, dans lequel les informations relatives à une capacité à se déplacer sont basées sur l'une ou plusieurs : d'informations relatives au terrain ; ou d'informations géologiques ;
dans lequel au moins l'un des deux appareils ou plus comprend un drone (2756) agencé pour délivrer le produit à l'utilisateur (2750) et la sélection du drone (2756) pour délivrer le produit à l'utilisateur (2750) lorsque le déplacement de l'utilisateur (2750) vers un autre des deux appareils ou plus est déterminé comme étant difficile ou impossible, sur la base des informations relatives au terrain et/ou des informations géologiques ; et
dans lequel le produit comprend un produit de santé destiné au consommateur et la sélection du produit parmi une pluralité de produits de santé destinés au consommateur disponibles à l'aide d'un moteur de diagnostic (2531) pour déterminer un ou plusieurs symptômes médicaux de l'utilisateur (2750), dans lequel le moteur de diagnostic (2531) est compris dans l'un ou plusieurs : d'un serveur (2754) ; du dispositif utilisateur (2752) de l'utilisateur (2750).
